(19) Europäisches Patentamt
European Patent Office
Office européen des brevets

(11) Veröffentlichungsnummer : **0 639 644 A1**

(12) **EUROPÄISCHE PATENTANMELDUNG**

(21) Anmeldenummer : **94810460.9**

(22) Anmeldetag : **08.08.94**

(51) Int. Cl.[6] : **C12P 17/08,** C07D 313/00, C07D 407/06, A61K 31/335, A61K 31/365

(30) Priorität : **16.08.93 CH 2435/93**

(43) Veröffentlichungstag der Anmeldung : **22.02.95 Patentblatt 95/08**

(84) Benannte Vertragsstaaten : **AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Zeeck, Axel, Prof. Dr.**
**Brüder-Grimm-Allee 22**
**D-37075 Göttingen (DE)**
Erfinder : **Bindseil, Kai Uwe, Dr.**
**Rüttgasse 1**
**D-79618 Rheinfelden (DE)**
Erfinder : **Boddien, Claudia**
**Am Söhlenborn 9**
**D-37085 Göttingen (DE)**

(54) **Neue Makrolide und ihre Verwendung.**

(57) Verbindungen der Formel

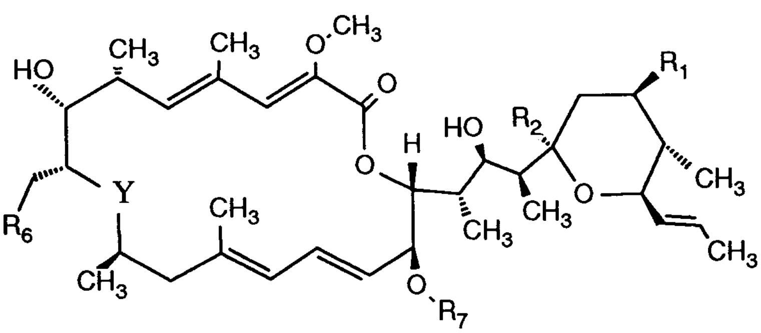

(I),

worin $R_1$ Hydroxy, 2-Hydroxyethoxy, $C_1$-$C_4$-Alkoxy, $C_1$-$C_4$-Alkanoyloxy, Halogen, Amin, Azid, eine Keto, p-Nitrobenzolsulfonyl oder eine Pyranyloxy-Gruppe der Formel

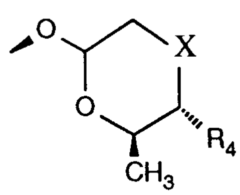

bedeutet, worin X eine Gruppe der Formel

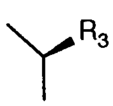 oder 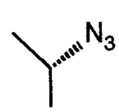

ist, worin $R_3$ für Hydroxy, $C_1$-$C_4$-Alkanoyloxy oder organisches Sulfonyloxy steht, und $R_4$ Hydroxy oder Carbamoyloxy ist,
$R_2$ Wasserstoff, Hydroperoxy, Hydroxy oder Methoxy darstellt, Y eine Gruppe der Formel

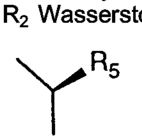 oder 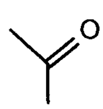

ist, worin $R_5$ Hydroxy oder $C_1$-$C_4$-Alkanoyloxy bedeutet, und $R_6$ und $R_7$ unabhängig voneinander

EP 0 639 644 A1

Jouve, 18, rue Saint-Denis, 75001 PARIS

Wasserstoff oder Methyl darstellt, sind zur Herstellung eines Arzneimittels für die Prophylaxe und Behandlung von Krankheiten, die auf die Inhibition der Osteoklasten-Protonenpumpe ansprechen, geeignet.

Die Erfindung betrifft neue Concanamycin-Verbindungen und die Verwendung von Concanamycin-Verbindungen zur prophylaktischen und therapeutischen Behandlung des menschlichen und tierischen Körpers.

Im menschlichen und tierischen Körper besteht ein dynamisches Gleichgewicht zwischen der kontinuierlichen Resorption alter Knochensubstanz durch Osteoklasten und der kontinuierlichen Knochenneubildung durch Osteoblasten. Die Knochenresorption und -neubildung ist im gesunden Organismus eng miteinander verknüpft, so dass die Gesamtknochenmasse konstant bleibt. Bei osteotropen Erkrankungen wie z.B. Osteopenie übersteigt die Resorption die Neubildung, woraus ein realer Verlust an Knochensubstanz resultiert. Osteoklasten-Zellen befinden sich in Lakunen auf der Oberfläche von Knochen und entfalten ihre resorbierende Aktivität dadurch, dass sie das extrazelluläre Kompartiment zwischen Knochen und Osteoklastenmembran durch Protonensekretion acidifizieren. Der resultierende niedrige pH-Wert führt zu einer Demineralisation des Knochens und bewirkt eine Aktivierung von lysosomalen Enzymen, welche in der Folge die freigesetzte Kollagenmatrix abbauen.

Untersuchungen über den für die Acidifizierung verantwortlichen Mechanismus haben gezeigt, dass ein in der Osteoklastenmembran lokalisiertes Enzym, eine ATP-abhängige Protonenpumpe (oder $H^+$-ATPase), speziell die Osteoklasten-Protonenpumpe, für die Entstehung und Aufrechterhaltung des pH-Gradienten durch diese Membran eine entscheidende Rolle spielt. Die Osteoklasten-Protonenpumpe ist ein Mitglied der multimeren vakuolären Klasse der $H^+$-ATPase-Enzyme, die in der Membran einer Vielzahl von intracellulären Vakuolen (z.B. Lysosomen, Clathrin-beschichtete Vesikel und chromaffine Granula) anzutreffen sind. Die Inhibierung der Osteoklasten-Protonenpumpe und folglich des Acidifizierungsprozesses stellt einen Weg dar, den Verlust an Knochensubstanz bei Osteoporose und anderen Osteopenie-Erkrankungen zu unterbinden.

Es ist ein Gegenstand der vorliegenden Anmeldung, Inhibitoren der Osteoklasten-Protonenpumpe und damit für die Behandlung von Erkrankungen, welche durch einen Verlust an Knochensubstanz gekennzeichnet sind, geeignete pharmazeutische Mittel bereitzustellen.

Von den Concanamycinen A, B und C, aus Streptomyces spec. erhältlichen Makrolid-Antibiotika, ist bekannt, dass sie potente Inhibitoren der vakuolären $H^+$-ATPase-Enzyme sind (S. Dröse et al., Biochemistry 32 (1993) 3902-3906). Da die vakuolären $H^+$-ATPase-Enzyme für zahlreiche intrazelluläre Funktionen lebensnotwendig sind, würde man von Inhibitoren dieser Enzymklasse eine hohe Cytotoxizität erwarten. Überraschenderweise wurde jetzt gefunden, dass bestimmte Concanamycin-Verbindungen hochaktive und äusserst spezifische Inhibitoren der Osteoklasten-Protonenpumpe ("OPPI's") sind, d.h. OPPI's ohne messbare Cytotoxizität (vakuoläre $H^+$-ATPase-Enzyme werden weniger tangiert) und mit grossem therapeutischen Fenster. Die bekannten Concanamycine A, B und C sind durch die folgende Struktur gekennzeichnet:

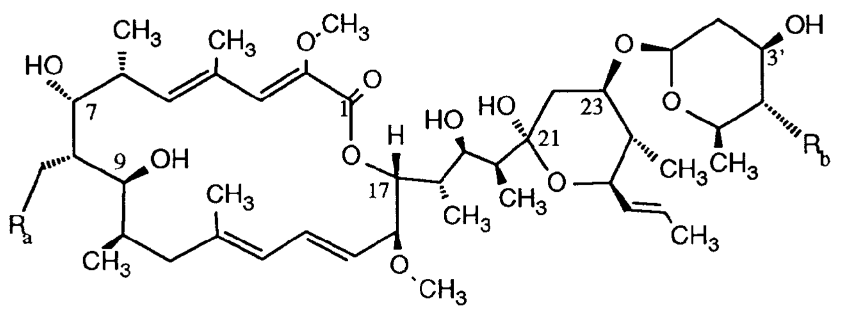

Concanamycin A: $R_a$= $CH_3$; $R_b$= O-C(=O)-$NH_2$
Concanamycin B: $R_a$= H; $R_b$= O-C(=O)-$NH_2$
Concanamycin C: $R_a$= $CH_3$; $R_b$= OH.

Die Erfindung betrifft die Verwendung von Verbindungen der Formel

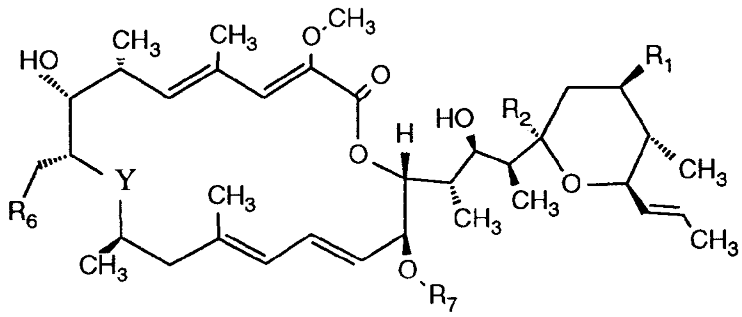 (I),

worin $R_1$ Hydroxy, 2-Hydroxyethoxy, $C_1$-$C_4$-Alkoxy, $C_1$-$C_4$-Alkanoyloxy, Halogen, Amin, Azid, Keto, p-Nitrobenzolsulfonyl oder eine Pyranyloxy-Gruppe der Formel

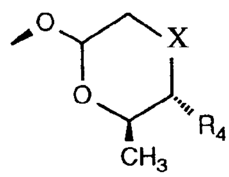

bedeutet, worin X eine Gruppe der Formel

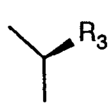 oder 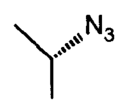

ist, worin $R_3$ für Hydroxy, $C_1$-$C_4$-Alkanoyloxy oder organisches Sulfonyloxy steht, und $R_4$ Hydroxy, $C_1$-$C_4$-Alkanoyloxy oder Carbamoyloxy ist,
$R_2$ Wasserstoff, Hydroperoxy, Hydroxy, $C_1$-$C_4$-Alkoxy; bevorzugt Wasserstoff, Hydroxy oder Methoxy, darstellt, Y eine Gruppe der Formel

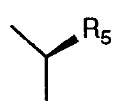 oder 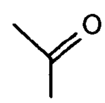

ist, worin $R_5$ Hydroxy oder $C_1$-$C_4$-Alkanoyloxy bedeutet, und
$R_6$ und $R_7$ unabhängig voneinander Wasserstoff oder bevorzugt Methyl darstellt, zur Herstellung eines Arzneimittels, welches für die Prophylaxe und Behandlung von Krankheiten, die auf die Inhibition der Osteoklasten-Protonenpumpe ansprechen, geeignet ist.

$C_1$-$C_4$-Alkoxy ist z.B. Ethoxy oder insbesondere Methoxy. $C_1$-$C_4$-Alkanoyloxy ist z.B. Formyloxy, Propionyloxy oder insbesondere Acetoxy. Organisches Sulfonyloxy ist z.B. $C_1$-$C_4$-Alkansulfonyloxy, wie Methansulfonyloxy, oder unsubstituiertes oder durch $C_1$-$C_4$-Alkyl, wie Methyl, Halogen, wie Chlor oder Brom, oder Nitro einfach substituiertes Benzolsulfonyloxy, z.B. 4-Nitrobenzolsulfonyloxy.

Ist $R_1$ eine Keto-Gruppe so stellt die Bindung zwischen $C_{23}$ und dem Sauerstoff eine Doppelbindung dar.

Der Ausdruck Halogen steht für Fluor, Chlor, Brom oder Jod, insbesondere für Chlor oder Brom.

Bevorzugt ist die Verwendung von solchen Verbindungen der Formel (I), worin $R_1$ Hydroxy, Methoxy, $NH_3$, Chlor, Brom, eine Keto, p-Nitrobenzolsulfonyl oder eine Pyranyloxy-Gruppe der Formel

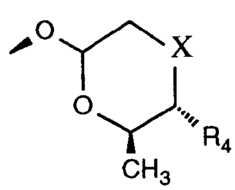

bedeutet, worin $R_4$ Hydroxy, Acetoxy oder Carbamoyloxy ist und X eine Gruppe der Formel

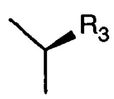

oder

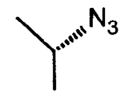

ist, worin $R_3$ für Hydroxy, Acetoxy oder unsubstituiertes oder durch $C_1$-$C_4$-Alkyl, Halogen oder Nitro einfach substituiertes Benzolsulfonyloxy steht, $R_2$ Wasserstoff oder Hydroxy darstellt, Y eine Gruppe der Formel

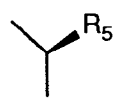

oder

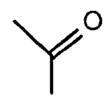

ist, worin $R_5$ Hydroxy oder Acetoxy bedeutet und $R_6$ Methyl darstellt.

Besonders bevorzugt ist die Verwendung von solchen Verbindungen der Formel (I), worin $R_1$ Hydroxy, Methoxy, Azid, Chlor, eine Keto, p-Nitrobenzolsulfonyl oder eine Pyranyloxy-Gruppe der Formel

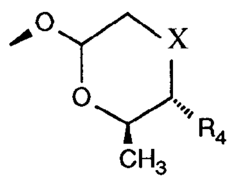

bedeutet, worin $R_4$ Hydroxy oder Carbamoyloxy ist und X eine Gruppe der Formel

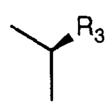

ist, worin $R_3$ Hydroxy oder Acetoxy bedeutet, $R_2$ Wasserstoff oder Hydroxy darstellt, und Y eine Gruppe der Formel

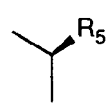

darstellt und $R_5$ Hydroxy oder Acetoxy bedeutet.

Insbesondere betrifft die Erfindung die Verwendung von Concanamycin A, Concanamycin C, Concanolid A, 21-Desoxy-concanolid A, 23-O-Methyl-concanolid A, 3',9-Di-O-acetyl-concanamycin A, 16-Desmethyl-21-desoxyconcanolid A, 21,23-Di-desoxy-23-epi-azidoconcanolid A, 23-O-(p-Nitrobenzolsulfonyl)-21-desoxyconcanolid A, 21-Desoxyconcanolid A-23-keton, 21-Desoxyconcanolid A-9, 23-diketon, Desoxyconcanamycin A und/oder 21,23-Didesoxy-23-epi-chlorconcanolid A.

Die bekannten und neuen Verbindungen der Formel (I) besitzen wertvolle pharmakologische Eigenschaften und können für die Prophylaxe und Therapie von Krankheiten, die auf die Inhibition der Osteoklasten-Protonenpumpe ansprechen, eingesetzt werden. Bei derartigen Krankheiten, die durch einen erhöhten Verlust an Knochensubstanz gekennzeichnet sind, handelt es sich z.B. um primäre (juvenile, idiopathische, kongenitale, postmenopausale (Typ 1) und involutionale (Typ 2)) Osteoporose und sekundäre (mit malignen Tumoren, renaler Insuffizienz, Endokrinopathien, Malabsorptionssyndrom, Lebererkrankungen, Immobilisation, Arthropathien und iatrogenen Krankheiten verbundene oder durch diese Krankheiten verursachte) Osteopenie.

Zur Feststellung der inhibierenden Wirkung können Clathrin-beschichtete Vesikeln aus Rinderhirn (enthalten eine vakuoläre $H^+$-ATPase) als Enzymquelle benutzt werden (vgl. Xie et al. Methods in Enzym.(1988) 157, 634-646). Die Cytotoxizität der untersuchten Verbindungen wird mit konfluenten Kulturen von Maus 3T3-Fibroblasten und XTT Tetrazolium-Färbung bestimmt (nach Roehm et al. J. Immunol. Methods (1991) 142, 257-265). Die erfindungsgemäss verwendbaren Verbindungen sind potente Inhibitoren der $H^+$-ATPase-Protonenpumpe mit $IC_{50}$-Werten im submikromolaren Bereich (ca. 0,5-15 nanomolar) bei einer Cytotoxizität ($IC_{50}$) im mikromolaren Bereich (ca. 2-10 mikromolar). Das therapeutische Fenster der Verbindungen ergibt sich aus dem Vergleich der im $H^+$-ATPase-Enzym-Test und im Cytotoxizitätstest festgestellten Inhibierungswirkungen. Die erfindungsgemäss verwendbaren Verbindungen weisen ein Verhältnis $IC_{50}$ (μM, Cytotoxizitätstest): $IC_{50}$ (μM, $H^+$-ATPase-Enzym-Test) von grösser als 500 auf und besitzen damit eine hohe inhibierenden Wirkung auf vakuoläre $H^+$-ATPase-Enzyme bei niedriger Cytotoxizität. Die erfindungsgemäss verwendbaren Verbindungen inhibieren weiterhin die Knochenresorption im Maus-Calvaria-Test (vgl. Reynolds in: Ball et al. (1976), Organic Culture in Biomedical Research, Cambridge University Press, 355-366). Die $IC_{50}$-Werte im Maus-Calvaria-Test liegen bei <0,3 μM.

Die unter Verwendung der Verbindungen der Formel (I) hergestellten Arzneimittel liegen insbesondere in Form von pharmazeutischer Zusammensetzungen vor, die zur prophylaktischen und therapeutischen Behandlung des menschlichen oder tierischen Körpers, insbesondere im Falle der obengenannten Krankheiten, geeignet sind. Solche pharmazeutischen Zusammensetzungen enthalten eine wirksame Menge, insbesondere eine zur Prophylaxe oder Therapie einer der obengenannten Krankheiten wirksame Menge, der Aktivsubstanz zusammen mit pharmazeutisch verwendbaren Trägerstoffen, die sich zur topischen, enteralen, z.B. rektalen oder oralen, oder parenteralen, z.B. transdermalen, subkutanen oder intravenösen, Verabreichung eignen und anorganisch oder organisch, fest oder flüssig sein können. Bevorzugt ist die orale Darreichungsform. Bei der Prophylaxe und Therapie von Krankheiten, die auf eine Inhibition der Osteoklasten-Protonenpumpe ansprechen, in einem Warmblüter wird letzterem eine prophylaktisch oder therapeutisch wirksame, die Osteoklasten-Protonenpumpe hemmende Dosis einer Verbindung der Formel (I) verabreicht. Die Dosierung des Wirkstoffs und die Art der Behandlung (Administrationsart; kontinuierlich, intermittierend, zyklisch) hängt unter anderem von der Art der Krankheit, der Art und Grösse der zu behandelnden Spezies, der Abwehrlage des Organismus und der Applikationsweise ab. Beispielsweise verabreicht man an einen Warmblüter von etwa 70 kg Körpergewicht eine tägliche Dosis von etwa 0,5 mg bis etwa 1500 mg, hauptsächlich von etwa 5 mg bis etwa 500 mg, vorzugsweise von etwa 10 mg bis etwa 300 mg, einer Verbindung der Formel (I). Diese Gesamt-Tagesdosis wird vorzugsweise auf 1-3 tägliche Applikationen verteilt. Dabei liegt die Dosierung bei oraler Applikation eher im oberen Bereich der angegebenen Dosierungen.

Zur oralen Verabreichung verwendet man insbesondere Tabletten oder Gelatinekapseln, welche den Wirkstoff zusammen mit Verdünnungsmitteln, z.B. Lactose, Dextrose, Sukrose, Mannitol, Sorbitol, Cellulose und/oder Glycerin, und/oder Schmiermitteln, z.B. Kieselerde, Talk, Stearinsäure oder Salze davon, wie Magnesium- oder Calciumstearat, und/oder Polyethylenglykol, enthalten. Tabletten können ebenfalls Bindemittel, z.B. Magnesiumaluminiumsilikat, Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmacksstoffe und Süssmittel enthalten. Ferner kann man die pharmakologisch wirksamen Verbindungen der vorliegenden Erfindung in Form von parenteral verabreichbaren Zusammensetzungen oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Zusammensetzungen können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Zusammensetzungen,die, wenn erwünscht, weitere pharmakologisch wirksame Stoffe, wie Antibiotika, enthalten können, werden in an sich bekannter Weise, z.B. mittels konventioneller Misch-, Granulier-, Dragier-, Lösungs- oder Lyophilisierungsverfahren, hergestellt und enthalten von etwa 0,01 % bis 90 %, im Fall von Lyophilisaten bis 100 %, insbesondere von etwa 0,1 % bis etwa 50 %, in erster Linie zwischen 1 % und 30 %, des bzw. der Aktivstoffe, wobei eine Wirkstoffkonzentration unterhalb von 1% insbesondere für topisch zu applizierende Zusammensetzungen geeignet ist.

Die Erfindung betrifft weiterhin die Verbindungen der Formel

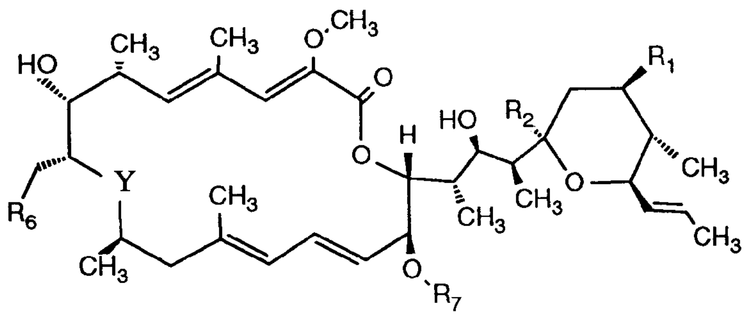

(I)

worin $R_6$ und $R_7$ unabhängig voneinander jeweils Wasserstoff oder bevorzugt Methyl ist und

- worin $R_1$ Halogen, Amin, Azid, Keto, p-Nitrobenzolsulfonyl oder Hydroxy ist, $R_2$ Wasserstoff bedeutet und Y eine Gruppe der Formel

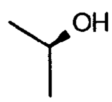

darstellt, oder

- worin $R_1$ Halogen, Amin, Azid, Keto, p-Nitrobenzolsulfonyl oder eine Pyranyloxy-Gruppe der Formel

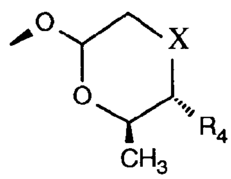

bedeutet, worin $R_4$ Carbamoyloxy ist und X eine Gruppe der Formel

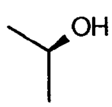

ist, $R_2$ für Hydroxy steht und Y eine Gruppe der Formel

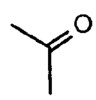

darstellt, oder

- worin $R_1$ Halogen, Amin, Azid, Keto, p-Nitrobenzolsulfonyl, 2-Hydroxyethoxy oder eine Pyranyloxy-Gruppe der Formel

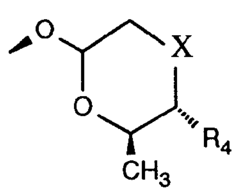

bedeutet, worin $R_4$ Hydroxy oder Carbamoyloxy ist und X eine Gruppe der Formel

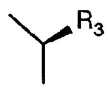

oder

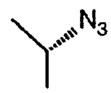

ist, worin $R_3$ für unsubstituiertes oder durch $C_1$-$C_4$-Alkyl, Halogen oder Nitro einfach substituiertes Benzolsulfonyloxy steht,
$R_2$ Wasserstoff, Hydroperoxy oder Hydroxy darstellt und Y eine Gruppe der Formel

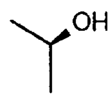

ist, oder

- worin $R_1$ Halogen, Amin, Azid, Keto, p-Nitrobenzolsulfonyl $C_1$-$C_4$-Alkoxy oder $C_1$-$C_4$-Alkanoyloxy ist, $R_2$ Hydroxy oder $C_1$-$C_4$-Alkoxy, bevorzugt Hydroxy oder Methoxy, darstellt und Y eine Gruppe der Formel

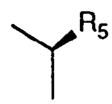

darstellt und $R_5$ $C_1$-$C_4$-Alkanoyloxy bedeutet,
und solche Verbindungen der Formel (I) enthaltende pharmazeutische Zusammensetzungen.

Besonders bevorzugt sind solche Verbindungen der Formel (I), worin

- $R_1$ Hydroxy, Azid, p-Nitrobenzolsulfonyl, eine Ketogruppe oder Chlor ist, $R_2$ Wasserstoff bedeutet und Y eine Gruppe der Formel

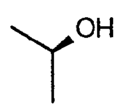

darstellt, oder

- $R_1$ eine Pyranyloxy-Gruppe der Formel

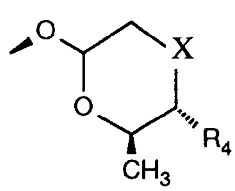

bedeutet, worin $R_4$ Carbamoyloxy ist und X eine Gruppe der Formel

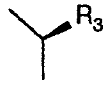

oder

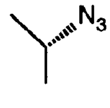

ist, worin $R_3$ durch Nitro einfach substituiertes Benzolsulfonyloxy ist, $R_2$ Hydroxy darstellt und Y eine Gruppe der Formel

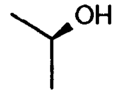

ist, oder

- $R_1$ Acetoxy ist, $R_2$ Hydroxy darstellt und Y eine Gruppe der Formel

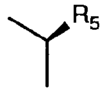

darstellt und $R_5$ Acetoxy bedeutet.

Insbesondere betrifft die Erfindung 21-Desoxy-concanolid A, 16-Desmethyl-21-desoxyconcanolid A, 21,23-Didesoxy-23-epi-azidoconcanolid A,
23-O-(p-Nitrobenzolsulfonyl)-21-desoxyconcanolid A, 21-Desoxyconcanolid A-23-keton, 21-Desoxyconcanolid A-9, 23-diketon, Desoxyconcanamycin A und/oder
21,23-Didesoxy-23-epi-chlorconcanolid A.

Die Verbindungen der Formel (I) sind bekannt oder können nach an sich bekannten Verfahren hergestellt werden. Die erfindungsgemäss verwendbaren und auch als Ausgangsstoffe zur Herstellung neuer erfindungsgemässer Verbindungen der Formel (I) benötigten Concanamycine A, B und C sind beispielsweise gemäss dem von Kinashi et al. beschriebenen Verfahren (J.Antibiotics (1984) 37, 1333-1343) durch Fermentierung von Streptomyces diastatochromogenes S-45 (oder auch Streptomyces spec. Gö 22/15, DSM 8426) und Isolierung aus dem Mycelium erhältlich.

Verbindungen der Formel (I) mit einer Ketofunktion in Position 9 (Y stellt eine Carbonylgruppe dar) können beispielsweise über das 3',9-Diketon hergestellt werden. Letzteres ist aus z.B. Concanamycin A durch (wegen der hohen Hydrolyselabilität) schonende Oxidation, z.B. mit dem Tetra-n-propylammonium-tetra-oxo-ruthenat(VII)-Reagenz (TPAP), erhältlich. Unter schonenden Bedingungen, z.B. mit Natriumborhydrid, lässt sich die 3'-Ketogruppe im Diketon selektiv reduzieren.

Die Acylierung freier Hydroxygruppen in den Positionen 9, 23 und/oder 3' erfolgt ebenfalls in an sich bekannter Weise mittels eines $C_1$-$C_4$-Alkansäureanhydrids (zur Einführung einer Acetylgruppe beispielsweise mit Acetanhydrid) in Pyridin. Die Umsetzung von Concanamycin A oder B mit Acetanhydrid ergibt so das 9,3'-Di-O-acetylderivat, von Concanamycin C ausgehend entsteht das 9,3'-Di-O-acetylderivat und das 9,3',4'-Tri-O-acetylderivat. In den deglykosylierten Concanoliden A und B ($R_1$ ist Hydroxy) ist auf gleiche Weise das 9,23-Di-O-acetylderivat erhältlich. Wenn eine der beiden Hydroxygruppen in den Positionen 9 und 23 z.B. durch eine Etherfunktion blockiert ist, wird die freie Hydroxygruppe unter Bildung des Mono-O-acetylderivats verestert.

Die 3'-Hydroxygruppe in den Concanamycinen A oder C kann durch Umsetzung mit entsprechenden Sulfonylchloriden selektiv O-sulfoniert werden. In einer Folgereaktion kann die 3'-Sulfonyloxy-Verbindung durch Umsetzen mit einem Azid, z.B. Natriumazid, in die 3'-Epiazido-Verbindung überführt werden.

Zur Herstellung von 21-$C_1$-$C_4$-Alkylderivaten der Concanamycine setzt man letztere mit den entsprechenden $C_1$-$C_4$-Alkanolen in Gegenwart einer Säure, vorzugsweise Lewis-Säure wie $FeCl_3$, um. Solche 21-$C_1$-$C_4$-Alkylderivate der Concanamycine können unter sauren Bedingungen (d.h. in Gegenwart von z.B. Salzsäure) mit Natriumcyanoborhydrid in die entsprechenden 21-Desoxyderivate umgewandelt werden.

Zur Deglykosylierung der Concanamycine (und Herstellung der Concanolide, $R_1$ ist Hydroxy) werden letztere mit einer organischen Sulfonsäure, z.B. p-Toluolsulfonsäure, gegebenenfalls in Gegenwart von Salzsäure in einem wässrigen organischen Lösungsmittel (wie wasserhaltiges Dimethylsulfoxid oder Acetonitril) umgesetzt.

Verbindungen der Formel (I), worin $R_1$ $C_1$-$C_4$-Alkoxy oder 2-Hydroxyethoxy bedeutet, können z.B. hergestellt werden, in dem man ein Concanamycin einer säurekatalysierten Alkanolyse, z.B. Methanolyse oder Ethandiolyse, unterwirft. Als Katalysator wird zweckmässig p-Toluolsulfonsäure eingesetzt. In Gegenwart von Lewis-Säuren sind so auch die 21,23-Dialkoxyderivate erhältlich.

Hinterlegung von Mikroorganismen

Der Stamm Streptomyces spec. Gö 22/15 wurde am 19.7.1993 bei der Deutschen Sammlung von Mikroorganismen und Zellkulturen (DSM) in Braunschweig (BRD) unter der Nummer DSM 8426 hinterlegt.

Die nachfolgenden Beispiele illustrieren die Erfindung, ohne sie in irgendeiner Form einzuschränken.

## Experimenteller Teil

### Allgemeine Methoden

Schmelzpunkte: Die Schmelz- und Zersetzungspunkte wurden mit einem Heiztischmikroskop der Fa. Reichert bestimmt und nicht korrigiert.

Massenspektren: EI-MS: Varian MAT 731, 70 eV; Finnigan MAT 311 A, 70 eV, Hochauflösungen mit Perfluorkerosin als Vergleichssubstanz; FAB-MS: Finnigan MAT 95 A (Matrix: $\alpha$-Nitro-benzylalkohol). DCI-MS: Finnigan MAT 95 A (Reaktandgas: $NH_3$)

Infrarotspektren: IR-Spektren wurden mit einem Spektrometer der Fa. Perkin-Elmer Modell 298 sowie einem FT-IR-Spektrometer der Fa. Perkin-Elmer Modell 1600 gemessen; alle Substanzen als KBr-Presslinge.

Elektronenspektren: Kontron Uvikon 860.

Drehwerte: Polarimeter Perkin-Elmer Modell 241, die Drehwerte sind in [$10^{-1}degcm^2g^{-1}$] angegeben, c in [$g100^{-1}$-$ml^{-1}$]

$^1$H-NMR-Spektren: Varian XL 200 (200 MHz), Varian VXR 200 (200 MHz), Varian VXR 500 (500 MHz), Bruker AMX 300 (300 MHz). Chemische Verschiebungen in $\delta_H$-Werten relativ zu Tetramethylsilan als internem Standard; Kopplungskonstanten (J) in Hz. Abkürzungen: s = Singulett, d = Duplett, t = Triplett, dd = Doppelduplett, m = Multiplett, br = breit.

$^{13}$C-NMR-Spektren: Varian XL 200 (50.3 MHz), Varian VXR 200 (50.3 MHz), Varian VXR 500 (125.7 MHz), Bruker AMX 300 (75.5 MHz). Chemische Verschiebungen in $\delta_C$-Werten relativ zu Tetramethylsilan als internem Standard.

Säulenchromatographie: Kieselgel 60 unter 0.08 mm (Macherey & Nagel); Kieselgel 60 0.2-0.05 mm (Macherey & Nagel); Kieselgel 60 0.04-0.063 mm (Macherey & Nagel); alle Kieselgelsäulen wurden mit p = 0.4-0.8 bar Überdruck betrieben (Flash-Chromatographie); Sephadex LH-20 (Pharmacia).

HPLC: Pumpe: Kontron 414, Detektor: Kontron 420, Mischkammer: Kontron M 800, Kontron Multiport, Datensystem: Kontron Data System 450; analytische 20 ml Aufgabeschleife, Vorsäule: Waters Guard Pak Precolumn Module 5 x 5 mm mit Resolve RP-18 Patronen; Säule: Kontron 10C18. System: isokratisches System, $CH_3CN$/Wasser 65:35, Flussrate 4 ml/min, Detektion 245 und 285 nm

Lösungsmittel und Chemikalien: Die Lösungsmittel für die Flashchromatographie wurden zuvor destilliert; für die HPLC wurden nur Lösungsmittel in Gradient Grade Qualität (Merck) und bidestilliertes Wasser, das vor Benutzung membranfiltriert wurde, verwendet. Für Reaktionen wurde im allgemeinen Gradient Grade Qualität (Merck) verwendet, die gegebenenfalls vorher über einem geeigneten Trockenmittel getrocknet wurden. Die eingesetzten Standard-Chemikalien wurden von Fluka, Aldrich und Merck bezogen und wie erhalten eingesetzt.

Laufmittelgemische für die Chromatographie:

| | |
|---|---|
| System A: $CHCl_3$/MeOH 8:2 | System B: $CHCl_3$/MeOH 9:1 |
| System C: $CHCl_3$/MeOH 95:5 | System D: EtOAc/n-Hexan 4:1 |
| System E: EtOAc/n-Hexan 2:1 | System F: EtOAc/n-Hexan 1:1 |
| System G: EtOAc/n-Hexan 1:2 | System H: EtOAc/n-Hexan 1:3 |
| System I: EtOAc/Benzol 4:1 | System K: MeOH/$H_2O$ 8:2 (auf RP-8) |

Sprühreagenzien: Nach Reagenzien Merck, Anfärbereagenzien für die Dünnschicht und Papierchromatographie.

Nährmediumsbestandteile: Alle Nährmediumsbestandteile wurden von der Fa. Difco bezogen.

Schüttler und Fermenter mit Belüftungseinstellung: Braun Certomat U, Braun Schüttelschrank BS4, Braun Biostat E (5 l; 5 l/min.), Braun Biostat E (15 l; 10 l/min.), Braun Biostat U (50 l; 2 $m^3$/h)

Aufarbeitung von Reaktionsgemischen: Unter der üblichen Aufarbeitung versteht sich die dreifache Extraktion mit dem angegebenen Lösungsmittel, Vereinigung der organischen Phasen, Trocknung über $Na_2SO_4$ und anschliessendes Einengen im Vakuum am Rotationsverdampfer.

Beispiel 1: Isolierung und Charakterisierung der Concanamycine A, B und C

a) Kultivierung von Streptomyces sp. (Gö 22/15) und Aufarbeitung

Die Stammhaltung erfolgt auf Petrischalen mit M2-Agar (10 g/l Malzextrakt, 4 g/l Hefeextrakt, 4 g/l Glucose, 25 g/l Agar). Die Platten werden von 48 h alten Schüttelkulturen bzw. von alten Platten beimpft und etwa 10 Tage bei 28°C inkubiert. Es bilden sich typische braun-gelbe Pocken. Das Luftmycel des Stammes ist weissgrau gefärbt. Schüttelkultur-Fermentationen erfolgen, wenn nicht anders angegeben, in mit 100 ml M2-Nährmedium gefüllten 250 ml Erlenmeyer-Kolben mit Schikane bzw. in mit 150 ml M2-Nährmedium gefüllten 1000 ml Erlenmeyer-Kolben. Zur Kultivierung wird mit Agarstücken ($1 cm^2$) überwachsener Platten angeimpft und 72 h bei 28°C geschüttelt. Als Vorkulturen für Bioreaktor-Fermentationen dienende Schüttelkulturen werden 48 h geschüttelt und anschliessend 24 h bei 4°C gekühlt.
Bioreaktor-Fermentationen erfolgen im 5 l bzw. 50 l Fermenter mit M2-Nährmedium (10 g/l Malzextrakt, 4 g/l Glucose, 4 g/l Hefeextrakt, 0.15 g/l Niax-Entschäumer). Im 5 l Fermenter wird bei 220 Rpm und einer Belüftung von 5 l/min fermentiert; im 50 l Fermenter bei 200 Rpm und 2 $m^3/h$. Zur Animpfung wird in allen Fällen 10 Vol% 48 h alte gekühlte Vorkultur herangezogen. Die Fermentation erfolgt 72 h in einem pH-Bereich von 5.8-7.4, zu dessen automatischer Regulierung 2N Citronensäure und 2N NaOH verwendet werden. Zum Abschluss der Fermentation ist im allgemeinen ein pH-Wert von 7.4 und starker Citronensäureverbrauch zu beobachten. Liegt der pH-Wert nach 72 h unter 7.0, verlängert man die Fermentation um 24 h.
Der pH-Wert bei der Ernte schwankt zwischen 6.8 und 7.4 und wird mit 0.5N NaOH auf 7.4 eingestellt. Nach Zugabe von 20 Vol% Celite lässt sich das Mycel abtrennen. Der Filterkuchen wird in Aceton aufgenommen und die Zellen werden durch 15 minütige Behandlung im Ultraschallbad oder mit dem Ultraturrax (bei Fermentation im 50 l Masstab) aufgeschlossen. Man filtriert erneut und dampft das organische Lösungsmittel im Vakuum ab. Nach Wiederholen des Vorgangs werden die wässrigen Mycelextrakte vereinigt und 2-3 mal mit EtOAc extrahiert. Das Kulturfiltrat wird nur in Ausnahmefällen 2-3 mal mit EtOAc ausgeschüttelt.

b.) Isolierung der Concanamycine aus Streptomyces Gö 22/15 zur spektroskopischen Charakterisierung und für biologische Tests

Die Isolierung erfolgt aus 8.06 g Mycel-Extrakt einer 50 l-Fermentation nach dem folgenden Schema:

- Säulenchromatographie an Kieselgel (Gradient $CHCl_3$/Methanol 100:0 bis 9:1),
- Concanamycin C abtrennen und weiter reinigen Säulenchromatographie an Sephadex LH20 (Methanol),
- Concanamycine A und B abtrennen und mit MPLC an RP-18 Kieselgel ($CH_3CN$/Wasser 65:35) auftrennen.

Bereits die erste Chromatographie an Kieselgel (Gradient: $CHCl_3$-System C-System B, 8 x 60 cm) führt zu chromatographisch und spektroskopisch hoch angereicherten Concanamycin-Fraktionen. Für die spektroskopische Charakterisierung der Concanamycine werden 120 mg Concanamycin C einer Gelfiltration an Sephadex LH-20 (MeOH, 3 x 80 cm) unterworfen, nach der 109 mg Concanamycin C als weisser amorpher Feststoff anfällt. Ein 113 mg der homologen Concanamycine A und B enthaltendes Gemisch wird durch Mitteldruck-Chromatographie an RP-18 Kieselgel (4 x 60 cm, $CH_3CN$-$H_2O$ 65:35) in 84 mg Concanamycin A und 13 mg Concanamycin B getrennt.

Concanamycin A: Schmp. 163°C
$C_{46}H_{75}NO_{14}$ (866,10)
$R_f$: 0.19 (RP-18, $CH_3CN$-$H_2O$ 65:35); 0.31 (System B); 0.25 (System I)
$R_t$: 19.1 min (Standard HPLC-System)
FAB-MS (negative Ionen): m/e = 865 $[M^-]$
FT-IR: $\nu$ = 3430, 2960, 2880, 1710, 1690, 1620 w, 1450, 1380, 1250, 1110, 1070, 1030, 970, 920, 770 $cm^{-1}$
$[\alpha]_D^{20}$ = - 18 (c = 0.5, MeOH)

Concanamycin B: Schmp. 156°C
$C_{45}H_{73}NO_{14}$ (851.51)
$R_f$: 0.25 (RP-18, $CH_3CN$-$H_2O$ 65:35); 0.31 (System B); 0.25 (System I)
$R_t$: 14.1 min (Standard HPLC-System)
FAB-MS (negative Ionen): m/e = 851 $[M^-]$
FAB-MS (positive Ionen): m/e = 875 $[M^+ + Na]$
DCI-MS (positive Ionen): m/e = 852 (100%, $M^+$); 861 (75%); 870 (26%, $M+NH_4^+$)
$[\alpha]_D^{20}$= - 18 (c = 0.5, MeOH)

Concanamycin C: Schmp. 153°C
$C_{45}H_{74}O_{13}$ (823,07)

$R_f$: 0.35 (System B); 0.25 (System I)
$R_t$: 16.8 min (Standard HPLC-System)
FAB-MS (negative Ionen): m/e = 822 [$M^-$]
FT-IR: $\nu$ = 3430, 2960, 2880, 1690, 1620 w, 1450, 1380, 1250, 1110, 1070, 1030, 970, 920, 760 $cm^{-1}$
$[\alpha]_D^{20}$ = - 17 (c = 0.5, MeOH).

Beispiel 2: 21-O-Methyl-concanamycin A

In einem ausgeheizten 50 ml Kolben löst man 800 mg (0.92 mmol) Concanamycin A in 19 ml trockenem MeOH. Anschliessend werden 15 mg $FeCl_3$ (0.09 mmol, 0.1 Äquiv.) in 1 ml MeOH zugegeben und man lässt die Mischung 15 min bei Raumtemperatur rühren. Nach Zugabe von 20 ml Phosphat-Puffer (pH = 7, Merck) wird die Lösung in einen 250 ml Kolben überführt und das organische Lösungsmittel vorsichtig im Vakuum entfernt. Der verbleibende wässrige Rückstand wird mit $CHCl_3$ extrahiert und nach der üblichen Aufarbeitung an Kieselgel (System D, 4 x 10) filtriert. Dabei erhält man die Titelverbindung. Schmp. 141°C
$C_{47}H_{77}NO_{14}$ (880,13)
$R_f$: 0.31 (System B); 0.58 (System I).
FAB-MS (positive Ionen): m/e = 904 [$M^+$+Na] (5%); 665 (100%)
FAB-MS (negative Ionen): m/e = 1034 [$M^-$+matrix] (40%); 880 [$M^-$]
UV (MeOH): $\lambda_{max}$ ($\epsilon$)= 245 (37 000); 283 nm (17 000)
$[\alpha]_D^{20}$ = + 45.6 (c = 0.4, MeOH)

Beispiel 3: 9,3'-Di-O-acetyl-concanamycin A

Eine Lösung von 78 mg Concanamycin A in 0.8 ml Pyridin wird mit 0.7 ml Acetanhydrid versetzt und 3 h bei Raumtemperatur gerührt. Wenn kein Edukt mehr zu detektieren ist (DC-Kontrolle), wird die Reaktionsmischung in 10 ml Eiswasser getropft und 30 min hydrolysiert. Nach Extraktion der resultierenden Mischung mit $CHCl_3$ und anschliessender Standard-Aufarbeitung wird das erhaltende Rohprodukt mehrfach in Toluol aufgenommen, um Pyridin und Essigsäure azeotrop abzudestillieren. Säulenchromatographische Aufreinigung des Produkts an Kieselgel (System E, 3 x 30 cm) liefert die Titelverbindung.
$C_{50}H_{79}NO_{16}$ (950,17)
$R_f$: 0.69 (System C) und 0.63 (System E).
FAB-MS (positive Ionen): m/e = 972 [$M^+$+Na]
FAB-MS (negative Ionen): m/e = 948 [$M^-$]
$[\alpha]_D^{20}$= -28 (c = 0.5, $CHCl_3$).

Beispiel 4: 3'-O-(p-Nitro-benzylsulfonyl)-concanamycin A

Zu einer Lösung von 50 mg (0.058 mmol) Concanamycin A in 5 ml $CH_2Cl_2$ werden nacheinander 35 mg DMAP (0.29 mmol, 5 Äquiv.), 0.5 ml Triethylamin und 130 mg p-Nitrobenzol-4-sulfochlorid (0.58 mmol, 10 Äquiv.) zugegeben. Die Reaktionsmischung wird 2 h bei Raumtemp. gerührt. Nach vollständiger Umsetzung wird die Reaktion durch Zugabe von Eis gestoppt, die Phasen werden getrennt und die wässrige Phase mit $CH_2Cl_2$ extrahiert. Die übliche Aufarbeitung und nachfolgende SC an Kieselgel (System G, 3 x 40 cm) liefert die Titelverbindung als gelbes Öl, die mit etwa 10% des instabilen 3',9-Di-O(p-Nitro-benzylsulfonyl)-concanamycin A verunreinigt ist.
$C_{52}H_{78}N_2O_{18}S$ (1051,25)
$R_f$: 0.72 (System B); 0.93 (System E).
FAB-MS (negative Ionen): m/e = 1051 [$M^-$]
IR: $\nu$ = 3450, 2980, 1720, 1690, 1610, 1530 (-$NO_2$), 1350 (-$SO_2$-O-), 1190 (-$SO_2$-O-), 1080 $cm^{-1}$
UV (MeOH): $\lambda_{max}$ ($\epsilon$) = 245 (41 000); 284 nm (17 000)
$[\alpha]_D^{20}$ = - 38 (c = 0.3, $CHCl_3$).

Beispiel 5: 3'-Desoxy-epiazido-concanamycin A

Eine Mischung von 96 mg (0.092 mmol) 3'-O-(p-Nitro-benzylsulfonyl)-concanamycin A (Beispiel 4) und 120 mg $NaN_3$ (1.84 mmol, 20 Äquiv.) in 10 ml DMF wird 5 h bei 45°C gerührt. Nach Zugabe von 15 ml Phosphatpuffer (pH = 7) wird die Lösung mit $CHCl_3$ extrahiert. Nach der üblichen Aufarbeitung wird mehrfach in Toluol aufgenommen, um restliches DMF durch azeotrope Destillation (25 Torr, 50°C) im Rotationsverdampfer zu entfernen. Die anschliessende Reinigung des Rohprodukts durch SC an Kieselgel (System E, 3 x 30 cm) ergibt

die Titelverbindung als blassgelben amorphen Feststoff. Schmp. 123°C.
$C_{46}H_{74}N_4O_{13}$ (891,11).
$R_f$: 0.74 (System B); 0.74 (System F); 0.18 (System G)
FAB-MS (negative Ionen): m/e = 1144 [$M^-$ + matrix]; 890 [$M^-$]
IR: $\nu$ = 3450, 2980, 2110 (-$N_3$), 1720, 1690, 1620 w, 1250, 1110, 1090 $cm^{-1}$
UV (MeOH): $\lambda_{max}$ ($\in$) = 245 (37 000); 284 nm (16 500)
$[\alpha]_D^{20}$ = -17 (c = 0.6, MeOH)
$^{13}$C-NMR (500 MHz, $CDCl_3$-$CD_3OD$ 2:1, -20°C): d = 6.9 (20-Me); 9.3 (18-Me); 11.7 (10-Me); 13.3 (24-Me); 14.2 (4-Me); 16.2 (12-Me); 16.9 (6-Me); 17.7, 17.8 (C-28 und 6-Me); 21.8, 22.7 (8-Et); 35.0 (C-6); 35.9 (C-10); 36.3 (C-2 ); 37.0 (C-18); 39.6 (C-22); 41.40, 41.44 (C-20 und C-24); 43.7 (CD-8); 45.4 (C-11); 55.8 (16-OMe); 59.2, 59.3 (16-OMe und C-3 ); 68.0 (C-5 ); 70.2 (C-19); 73.5 (C-7); 75.1, 75.5, 75.6, 75.9 (C-17, C-23, C-25 und C-4 ); 79.7 (C-9); 81.7 (C-16); 94.2 (C-1 ); 99.7 (C-21); 123.0 (C-13); 126.7 (C-15); 128.3 (C-27); 131.0 (C-26); 131.5, 132.0 (C-3 und C-4); 134.1 (C-14); 141.1, 141.7 (C-2 und C-5); 143.0 (C-12); 156.3 ($OCONH_2$); 166.9 (C-1) ppm.

Beispiel 6: 23-O-Methyl-concanolid A

Eine Lösung von 140 mg (0.17 mmol) Concanamycin C in 25 ml MeOH (p.A.) wird mit 20 mg (0.10 mmol, 0.6 Äquiv.) Toluol-4-sulfonsäure Monohydrat (techn., umkristallisiert aus $CHCl_3$) versetzt und 3 h bis zur vollständigen Umsetzung (DC-Kontrolle) bei Raumtemperatur gerührt. Die zunächst trübe Lösung wird dabei vollkommen klar. Die Mischung wird daraufhin in einen mit 5 ml Phosphatpuffer (pH = 7) gefüllten 250 ml Kolben überführt, das organische Lösungsmittel am Rotationsverdampfer im Vakuum vorsichtig entfernt und der wässrige Rückstand mit $CHCl_3$ extrahiert. Die übliche Aufarbeitung, gefolgt von einer SC an Kieselgel (System G, 3 x 30 cm), ergibt die Titelverbindung.
$C_{40}H_{66}O_{10}$ (706,96)
$R_f$: 0.62 (System C); 0.44 (System G)
FAB-MS (negative Ionen): m/e = 707 [$M^-$]
IR: $\nu$ = 3460, 2960, 1690, 1610w; 1440, 1350, 1240, 1100 $cm^{-1}$
UV (MeOH): $\lambda_{max}$ ($\in$) = 245 (40 000); 283 nm (18 000)
$[\alpha]_D^{20}$ = + 16 (c = 0.3, $CHCl_3$).

Beispiel 7: 21,23-Di-O-methyl-concanolid A

Eine Lösung von 140 mg (0.17 mmol) Concanamycin C wird entsprechend der allgemeinen Arbeitsvorschrift zur Methanolyse der Concanamycine (vgl. Beispiel 6), jedoch mit Chemikalien technischer Qualität, umgesetzt. Vollkommen analoge Aufarbeitung gefolgt von einer SC an Kieselgel (System G, 3 x 30 cm) ergibt die Titelverbindung als weissen amorphen Feststoff. Schmp. 97°C.
$C_{41}H_{68}O_{10}$ (720,98)
$R_f$: 0.53 (System C); 0.50 (System G).
FAB-MS (positive Ionen): m/e = 745 [$M^+$+Na]
FAB-MS (negative Ionen): m/e = 721 [$M^-$]; 773 [$M^-$+matrix]
IR: $\nu$ = 3480, 2960, 1690, 1610w; 1440, 1350, 1250, 1100 $cm^{-1}$
UV (MeOH): $\lambda_{max}$ ($\in$) = 245 (42 000); 283 nm (18 000)
$[\alpha]_D^{20}$ = + 9 (c = 0.3, $CHCl_3$)
$^{13}$C-NMR (125 MHz, $CH_2Cl_2$-$CD_3OD$ 2:1, -20°C): d = 7.5 (20-Me); 10.5 (18-Me); 12.1 (8-Et); 13.4 (24-Me); 14.3 (4-Me); 16.4 (12-Me); 17.1 (6-Me); 18.0 (C-28); 34.5, 35.0, 35.4, 35.9 (C-6, C-10, C-18, C-22); 38.3, 41.3 (C-20 und C-24); 44.3 (C-8); 46.0 (C-11); 46.8 (21-OMe); 56.0 (16-OMe); 56.7 (23-OMe); 60.3 (2-OMe); 70.0 (C-19); 73.6 (C-7); 76.3, 77.3, 79.8, 79.9 (C-9, C-17, C-23, C-25); 82.8 (C-16); 104.2 (C-21); 123.3 (C-13); 127.0 (C-15); 130.5, 130.7, 131.8, 132.6 (C-3, C-4, C-26, C-28); 134.3 (C-14); 141.9, 142.1 (C-2 und C-5); 143.4 (C-12); 167.0 (C-1) ppm.

Beispiel 8: 9-O-Acetyl-21,23-di-O-methyl-concanolid A

37 mg (0.051 mmol) 21,23-Di-O-methyl-concanolid A werden entsprechend der allgemeinen Arbeitsvorschrift zur Acetylierung der Concanamycine (vgl. Beispiel 3) umgesetzt. Nach SC an Kieselgel (System G, 2 x 20 cm) erhält man die Titelverbindung.
$C_{43}H_{70}O_{11}$ (763,02).
$R_f$: 0.56 (System G)

FAB-MS (positive Ionen): m/e = 787 [$M^+$+Na]
FAB-MS (negative Ionen): m/e = 763 [$M^-$]
IR: $\nu$ = 3420, 2960, 1740, 1690, 1620w; 1450, 1370, 1250, 1100; 1010cm$^{-1}$
UV (MeOH): $\lambda_{max}$ ($\epsilon$) = 245 (42 000); 283 nm (21 000)
$[\alpha]_D^{20}$ = + 5 (c = 0.5, $CHCl_3$).

Beispiel 9: 23-O-(2-Hydroxyethyl)-concanolid A

700 mg (0.81 mmol) Concanamycin A werden in einem Gemisch aus 50 ml 1,2-Ethandiol, 10 ml THF und 0.2 ml 0.5 N HCl gelöst und bei Raumtemperatur gerührt. Die Reaktion wird nach 18 h durch Zugabe von 10 ml Phosphat-Puffer (pH = 7) und 190 ml Wasser abgebrochen und nach der üblichen Aufarbeitung mit $CHCl_3$ extrahiert. SC an Kieselgel (System E, 4 x 40 cm) ergibt die Titelverbindung.
$C_{41}H_{68}O_{11}$ (736,98)
$R_f$: 0.43 (System B); 0.45 (System E)
IR: $\nu$ = 3450, 2930, 1720 sh, 1690, 1620 w, 1460, 1250, 1110 cm$^{-1}$
UV (MeOH): $\lambda_{max}$ ($\epsilon$) = 245 (35 000); 283 nm (16 000)
$[\alpha]_D^{20}$ = - 5 (c = 0.3, $CHCl_3$)

Beispiel 10: Concanolid A

Eine Lösung von 118 mg (0.143 mmol) Concanamycin C in 15 ml Acetonitril und 3.5 ml Wasser wird mit 95 mg (0.50 mmol, 3.5 Äquiv.) Toluol-4-sulfonsäure Monohydrat versetzt und 20 h bei Raumtemp. gerührt. Anschliessend wird die Mischung im Eisbad auf 0°C gekühlt, mit 30 ml gesättigter $NaHCO_3$-Lösung versetzt und rasch mit $CHCl_3$ extrahiert. Nach der üblichen Aufarbeitung kann die Titelverbindung durch SC an Kieselgel (System E, 3 x 30 cm) erhalten werden.
$C_{39}H_{64}O_{10}$ (692,93)
$R_f$: 0.29 (System C); 0.79 (System E); 0.17 (System F)
FAB-MS (negative Ionen): m/e = 693 [$M^-$]
IR: $\nu$ = 3460, 2960, 1690, 1620 w, 1250, 1100, 960 cm$^{-1}$
UV (MeOH): $\lambda_{max}$ ($\epsilon$) = 245 (32 000); 284 nm (17 000)
$[\alpha]_D^{20}$ = + 11 (C = 0.3, $CHCl_3$).

Beispiel 11: 9,21-Di-O-acetyl-concanolid A

Die Darstellung der Titelverbindung gelingt entsprechend der allgemeinen Arbeitsvorschrift zur Darstellung acetylierter Concanamycine (vgl. Beispiel 3) aus 48 mg (0.070 mmol) Concanolid A. Nach Reinigung des Rohprodukts durch Chromatographie an Kieselgel erhält man die reine Verbindung. Schmp. 125°C.
$C_{43}H_{68}O_{12}$ (777,01).
$R_f$: 0.54 (System G).
FAB-MS (positive Ionen): m/e = 799 [$M^+$+Na]
FAB-MS (negative Ionen): m/e = 776 [$M^-$]
IR: $\nu$ = 3450, 2980, 1740, 1690, 1620 w, 1250, 1110cm$^{-1}$
UV (MeOH): $\lambda_{max}$ ($\epsilon$) = 245 (35 000); 283 nm (16 000)
$[\alpha]_D^{20}$ = + 7 (c = 0.4, $CH_2Cl_2$).

Beispiel 12: 21-Desoxyconcanamycin A

Eine Lösung von 388 mg (0.44 mmol) 21-O-Methyl-concanamycin A und 193 mg $NaBH_3CN$ (3.08 mmol, 7 Äquiv.) in 50 ml EtOH wird mit 4 ml 0.5 N HCl versetzt und bei Raumtemperatur gerührt, bis DC-chromatographisch kein Edukt mehr nachzuweisen ist. Die DC-Kontrolle des Reaktionsverlaufs gestaltet sich aufgrund des ähnlichen chromatographischen Verhaltens von Edukt und Produkt schwierig; der Produkt-Spot ist jedoch mit Anisaldehyd/$H_2SO_4$ Sprühreagenz gelb-braun anfärbbar, während das Ausgangsmateriel blau-violett anfärbbar ist. Nach 3 h werden 60 ml Phosphatpuffer (pH = 7) zugegeben, EtOH im Vakuum entfernt und die wässrige Lösung mit $CHCl_3$ extrahiert. An die übliche Aufarbeitung schliesst sich eine Chromatographie an Kieselgel (System D, 4 x 40 cm) an. Schmp. 140°C.
$C_{46}H_{75}NO_{13}$ (850,10).
$R_f$ = 0.31 (System B); 0.54 (System D)
FAB-MS (positive Ionen): m/e = 872 [$M^+$+Na]

IR: ν = 3450, 2980, 1710, 1690, 1620 w, 1380, 1250, 1100 $cm^{-1}$
UV (MeOH): $\lambda_{max}$ (∊) = 245 (36 500); 283 nm (16 000)
$[\alpha]_D^{20}$ = - 46 (c = 1, MeOH)
$^1$H-NMR (300 MHz, $CDCl_3$): d = 1.58 (d br, J = 6, 3H, 28-$H_3$); 1.80 (s, 3H, 12-Me); 1.94 (s, 3H, 4-Me); 2.18 (ddd, J = 12, 5, 1.5, 1H, 22-$H_{eq}$); 2.70 (m, 1-H, 6-H); 3.22 (m obs, 1-H, 9-H); 3.22 (s, 3H, 16-OMe); 3.55 (s, 3H, 2-OMe); 3.74 (ddd, J = 11, 9, 5, 1H, 3 -H); 3.83 (dd, J = 9,9, 1H, 16-H); 4.28 (dd, J = 9,9, 1H, 3-H); 4.56 (dd, J = 10,2, 1H, 1-H); 4.88 (s, 2H, $NH_2$); 5.18 (d br, J = 10, 1H, 17-H); 5.21 (dd, J = 15,9, 1H, 15-H); 5.31 (ddq, J = 15, 8, 2, 1H, 26-H); 5.54 (dq, J = 15, 6.5, 1H, 27-H); 5.6-5.7 (s br, 1H, 5-H); 5.77 (d br, J = 10, 1H, 13-H); 6.35 (s, 1H, 3-H); 6.51 (dd, J = 15, 10, iH, 14-H) ppm.

Beispiel 13: 21-Desoxy-concanolid A

Eine Lösung von 100 mg 21-Desoxyconcanamycin A (0.117 mmol) in 15 ml MeOH wird mit 500 mg (2.6 mmol, 22.5 Äquiv.) Toluol-4-sulfonsäure in 3 ml 1 N HCl versetzt und 48 h bei Raumtemperatur gerührt. Nach Zugabe von 20 ml Phosphat-Puffer (pH = 7) wird das Lösungsmittel im Vakuum entfernt. Die standardisierte Aufarbeitung gefolgt von einer SC an Kieselgel (System E dann System D), liefert die Titelverbindung. Schmp. 104°C.
$C_{39}H_{64}O_9$ (676,93).
$R_f$ = 0.55 (System B); 0.61 (System E); 0.44 (System F)
FAB-MS (positive Ionen): m/e = 833 [$M^+$+matrix]; 699 [$M^+$+Na]
IR: ν = 3450, 2960, 1690, 1650 w, 1620 w, 1450, 1380, 1250, 1110 $cm^{-1}$
UV (MeOH): $\lambda_{max}$ (∊) = 245 (39 000); 282 nm (17 000)
$[\alpha]_D^{20}$ = - 23 (c = 0.6, MeOH)
$^1$H-NMR (200 MHz, $CDCl_3$): d = 1.62 (dd, J = 6, 1, 3H, 28-$H_3$); 1.83 (s br, 3H, 12-Me); 1.98 (s br, 3H, 4-Me); 2.78 (m, 1H, 6-H); 3.24 (s, 3H, 16-OMe); 3.58 (s, 3H, 2-OMe); 3.31 (dd, J = 10, 8, 1H, 25-H); 3.83 (dd, J = 9,9, 1H, H-16); 5.23(dd, J = 9, 1.5, 1H, 17-H); 5.25 (dd, J = 15, 10, 1H, 15-H); 5.33 (ddq, J = 15, 8, 1, 1H, 27-H); 5.57 (dq, J = 15, 6, 1H, 26-H); 5.80 (d, J = 10, 1H, 13-H); 6.38 (s, 1H, 3-H); 6.52 (dd, J = 15, 10, 1H, 14-H) ppm.

Beispiel 14: Concanamycin A-3',9-diketon

400 mg (0.462 mmol) Concanamycin A werden in 40 ml trockenem Methylenchlorid gelöst und mit 250 mg (4 Äquiv.) 4-Methylmorpholin-4-oxid Monohydrat (Fluka) und 40 mg (0.1 Äquiv.) Tetrapropylammonium-perruthenat (Fluka) versetzt. Die resultierende Mischung wird bei Raumtemperatur gerührt, wobei der zunächst grüne Farbton der Lösung allmählich in schwarz umschlägt. Nach vollständiger Umsetzung, spätestens jedoch nach 2 Stunden (die Katalysatoraktivität unterliegt starken Schwankungen), wird die Reaktionsmischung ohne weitere Aufarbeitung durch SC an Kieselgel (System E, 3 x 30 cm) getrennt. Man erhält so die Titelverbindung. Schmp. 170°C.
$C_{46}H_{71}NO_{14}$ (862,07).
$R_f$ = 0.54 (System B); 0.53 (System E)
FAB-MS (negative Ionen): m/e = 862 [$M^-$]
IR: ν = 3450, 2970, 1740, 1690, 1620 w, 1450, 1250, 1100 $cm^{-1}$
UV (MeOH): $\lambda_{max}$ (∊) = 245 (34 000); 283 nm (15 000)
$[\alpha]_D^{20}$ = - 52.0 (c = 0.9, $CH_2Cl_2$).

Beispiel 15: Concanamycin A-9-keton

Eine Lösung von 126.5 mg (0.146 mmol) Concanamycin A-3',9-diketon in 11 ml EtOH wird auf -78°C gekühlt. Nach Zugabe von 38.5 mg (7 Äquiv.) $NaBH_4$ wird 1 h unter Erhalt der Kühlung gerührt. Die Reaktion wird durch Zugabe von 10 ml Phosphatpuffer (pH = 7) zur noch kalten Mischung gestoppt und nach Erwärmen auf Raumtemperatur mit EtOAc extrahiert. Das nach üblicher Aufarbeitung erhaltene Rohprodukt wird durch SC (EtOAc - n-Hexan 3:1) gereinigt. Man erhält so die Titelverbindung. Schmp. 138°C.
$C_{46}H_{73}NO_{14}$ (864,08).
$R_f$ = 0.42 (System B); 0.24 (System E)
FAB-MS (negative Ionen): m/e = 863 [$M^-$]
IR: ν = 3450, 2970, 1740, 1690, 1620 w, 1450, 1250, 1100 $cm^{-1}$
UV (MeOH): $\lambda_{max}$ (∊) = 245 (34 000); 283 nm (15 000)
$[\alpha]_D^{20}$ = - 43.0 (c = 0.4, $CH_2Cl_2$).

Beispiel 16: Pharmazeutische Zusammensetzung

Tabletten enthaltend 20 mg an Wirkstoff, z.B. eine der in den vorangehenden Beispielen beschriebenen Verbindungen der Formel (I), werden in folgender Zusammensetzung in üblicher Weise hergestellt:

Zusammensetzung:

| | |
|---|---|
| Wirkstoff | 20 mg |
| Weizenstärke | 60 mg |
| Lactose | 50 mg |
| Kolloidale Kieselsäure | 5 mg |
| Talk | 9 mg |
| Magnesiumstearat | 1 mg |
| | 145 mg |

Herstellung;

Der Wirkstoff wird mit einem Teil der Weizenstärke, mit Lactose und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 17: 21-Desoxyconcanamycin A

Eine Lösung von 500 mg (0.57 mmol) 21-O-Methyl-concanamycin A und 258 mg $NaBH_3CN$ (4.11 mmol, 7 Äquiv.) in 65 ml EtOH wird mit 5 ml 0.5 N HCl versetzt und bei Raumtemperatur 4 h gerührt. Die DC-Kontrolle erfolgt über RP 8-Kieselgelplatten (Merck) (Laufmittel: MeOH/$H_2O$ 9:2). Zur Aufarbeitung werden 60 ml Phosphatpuffer (pH = 7) zugegeben, EtOH im Vakuum entfernt und der wässrige Rückstand mit $CHCl_3$ extrahiert. Die Aufreinigung erfolgt über MPLC-Chromatographie an RP 18-Kieselgel (Laufmittel MeOH/$H_2O$ 8:2). Die Ausbeute beträgt 228 mg (47 %) Smp: 140°C.
$R_f$ = 0.44 (System K)

Beispiel 18 und 19: 21-Desoxyconcanolid A und 16-Desmethyl-21-desoxyconcanolid A

Eine Lösung von 467 mg 21-Desoxyconcanamycin A (0.55 mmol) in 55 ml $CH_3CN/H_2O$ (5:1) wird mit 350 mg (1.83 mmol, 3 Äquiv.) Toluolsulfonsäure-monohydrat (Umkristallisiert aus $CHCl_3$) versetzt und bei 38°C 12 h gerührt. Zur Aufarbeitung wird auf 0°C gekühlt, mit 50 ml ges. $NaHCO_3$-Lsg versetzt, das organische Lösungsmittel im Vakuum entfernt und die wässrige Phase mit $CHCl_3$ extrahiert. DC-Kontrolle an Kieselgel (Laufmittel EtOAc/Hexan 1:1) zeigte neben dem Ausgangsmaterial am Startfleck zwei neue Zonen. Säulenchromatographie an Kieselgel (Gradient EtOAc/Hexan, 1:1 EtOAc) liefern 63 mg (22 % vom Umsatz) 16-Desmethyl-21-desoxyconcanolid A, 122 mg 21-Desoxyconcanolid A (42 % vom Umsatz) und 105 mg Ausgangsmaterial.

21-Deoxyconcanolid A:
Smp.: 122°C
$C_{39}H_{64}O_9$ (676.93)
$R_f$ = 0.40 (System F)
FAB-MS (positive Ionen): m/e = 699 [$M^+$ + Na]
IR: $\nu$ = 3450, 2960, 1690, 1650 w, 1620w, 1450, 1380, 1250, 1110 $cm^{-1}$

UV (MeOH): $\lambda_{max}$ (e) = 245(39000); 282 nm (17000)
$[\alpha]^{20}_D$ = - 23 (c = 0.6, MeOH)
$^1$H-NMR (500 MHz, $CD_2Cl_2/CD_3OD$ 2:1, -20°C): $\delta_H$ = 0.78-0.86 (m, 12 H, 20-Me, 18-Me, 8-Ethyl-$CH_3$, 24-Me); 0.98 (d, J = 7, 3 H, 6-Me); 1.02 (d, J = 6.5, 3 H, 10-Me); 1.09-1.18 (m, 4 H, 22β-H, 24 H, 8-Ethyl-$CH_2$); 1.42 (ddt, J = 11, 8, 3, 1 H, 8-H); 1.60 (dd, J = 6.5, 1.5, 3 H, 28-Me); 1.88 (s, 3 H, 12-Me); 1.94 (s, 3 H, 4-Me); 1.97-2.07 (m, 4 H, 11-$H_2$, 22α-H, 18-H); 2.38 (m, 1 H, 10-H); 2.67 (m, 1 H, 6-H); 3.12 (d, J = 10.5, 1 H, 9-H); 3.22 (s, 3 H, 16-OMe); 3.26-2.33 (m, 2 H, 23-H, 25-H); 3.43 (dt, J = 10, 0.5, 1 H, 21-H); 3.51 (s, 3 H, 2-OMe); 3.62 (dd, J = 10, 1, 1 H, 19-H); 3.67 (dd, J = 10, 2.5, 1 H, 7-H); 3.86 (dd, J = 9, 1 H, 16-H); 5.11 (dq, J = 9,0.5, 1 H, 17-H); 5.15(dd, J = 15, 9, 1 H, 15-H); 5.28 (ddq, J = 15, 8, 1, 1 H, 26-H); 5.56 (dq, J = 15, 6.6, 1 H, 27-H); 5.68 (d, J = 9.5, 1 H, 5-H); 5.76 (d, J = 11, 1 H, 13-H); 6.43 (s, 1 H, 3-H); 6.61 (dd, J = 15, 11, 1 H, 14-H) ppm.

16-Desmethyl-21-desoxyconcanolid A:
Smp.: 87°C
$C_{38}H_{62}O_9$ (662.90)
$R_f$ = 0.60 (System F)
FAB-MS (positive Ionen): m/e = 684 [$M^+$ + Na]
IR: ν = 3449, 2962, 2929, 1711, 1623, 1454, 1374, 1244, 1106, 1016, 970 $cm^{-1}$
UV (MeOH): $\lambda_{max}$ (e) = 243 (23200); 278 nm (13400)
$[\alpha]^{20}_D$ = - 108.61 (c = 0.86, MeOH)
$^1$H-NMR (500 MHz, $CD_2Cl_2/CD_3OD$ 2:1, -20°C): $\delta_H$ = 0.86 (m, 3H, 8-Ethyl-$CH_3$); 0.90 (d, J = 6.5, 3 H, 24-Me); 0.93 (d, J = 6.5, 3 H, 10-Me); 0.99 (d, J = 7, 3 H, 20-Me); 1.02 (d, J = 6.5, 3 H, 18-Me); 1.08 (d, J = 6.5, 3 H, 6-Me); 1.10 (m, verd, 8-Ethyl-$CH_2$, β-H); 1.15-1.24 (m, 3H, 24-H, 22β-H, 8-Ethyl-$CH_2$, α-H); 1.36 (m, 1 H, 8-H); 1.53 (dd, J = 16, 9, 1 H, 11β-H); 1.65 (s, 3 H, 12-Me); 1.71 (dd, J = 6.6, 1.5, 3 H, 28-Me); 1.79 (m, 1 H, 20-H); 1.86 (d, J = 18, 1 H, 11α-H); 1.92 (s, 3 H, 4-Me); 2.08 (dd, J = 14, 4, 1 H, 22α-H); 2.18 (m, 1 H, 18-H); 2.24 (m, 1 H, 10-H); 2.63 (m, 1 H, 6-H); 3.12 (dd, J = 10, 0.5, 1 H, 9-H); 3.28 (ddd, J = 12, 12, 5, 1 H, 23-H); 3.37 (m, 2 H, 21-H, 25-H); 3.56 (s, 3 H, 2-OMe); 3.57 (dd, J = 10, 2, 1 H, 7-H); 3.90 (dd, J = 9, 1 H, 9-H); 4.59 (dd, J = 9, 1 H, 16-H); 5.03 (dd, J = 9, 1.5, 1 H, 17-H); 5.38 (m, verd, 1 H, 26-H); 5.43 (dd, J = 15, 9, 1 H, 15-H); 5.69 (dq, J = 15, 6, 1 H, 27-H); 5.79 (d, J =11, 1 H, 13-H); 5.93 (d, J = 10, 1 H, 5-H); 6.30 (dd, J = 15, 11, 1 H, 14-H); 6.43 (s, 1 H, 3-H) ppm.

$^{13}$C-NMR (125 MHz, $CD_2Cl_2/CD_3OD$ 2:1, -20°C): $\delta_C$ = 8.7 (20-Me); 12.5 (10-Me); 13.5 (24-Me); 13.7 (18-Me); 14.6 (4-Me); 17.2 (8-$CH_2$-$\underline{C}H_3$) 18.1 (28-Me); 18.3 (6-Me); 19.3 (12-Me); 25.2 (8-$\underline{C}H_2$-$CH_3$); 35.4 (C-10); 36.0 (C-6); 39.1 (C-22); 39.3 (C-20); 39.8 (C-18); 42.8 (C-11); 44.0 (C-24); 45.7 (C-8); 60.2 (2-OMe); 73.6 (C-21); 77.1 (C-9); 77.3 (C-23); 79.1 (C-7); 79.3 (C-16); 81.8 (C-19); 82.5 (C-17);83.2 (C-25); 121.9 (C-13); 128.4 (C-15); 129.1 (C-16); 129.6 (C-27); 130.9 (C-26); 131.9 (C-3); 132.7 (C-4); 138.7 (C-12); 141.6 (C-5); 142.5 (C-2); 165.7 (C-1) ppm.

## Beispiel 20: 23-O-(p-Nitrobenzolsulfonyl)-21-desoxyconcanolid A

Zu einer Lösung von 85.5 mg 21-Deoxyconcanolid A (0.13 mmol) in 5 ml $CH_2Cl_2$ werden nacheinander 179.5 mg (1.5 mmol, 11.3 Äquiv.) DMAP, 1.6 ml Triethylamin und 334 mg p-Nitrobenzolsulfochlorid (1.5 mmol, 11.3 Äquiv.) zugegeben. Die Reaktionsmischung wird 1 h bei 0°C gerührt und durch Zugabe von Eis gestoppt. Die Phasen werden getrennt und die wässrige Phase mit $CHCl_3$ extrahiert. Das organische Lösungsmittel wird im Vakuum entfernt und das Reaktionsgemisch säulenchromatographisch an Kieselgel (Laufmittel EtOAc/Hexan 2:3) gereinigt. Ausbeute: 96.6 mg (85 %).
Smp.: 96°C
$C_{45}H_{67}O_{14}NS$ (878.09)
$R_f$ = 0.56 (System F)
FAB-MS (negative Ionen): m/e = 859 [$M^-$ $H_2O$]
IR: ν = 3485, 2967, 2875, 1696, 1534, 1351, 1248, 1186, 1102, 915 $cm^{-1}$
UV (MeOH): $\lambda_{max}$ (e) = 245 (55700); 278 nm (28200)
$[\alpha]^{20}_D$ = - 108.61 (c = 0.62, MeOH)
$^1$H-NMR (500 MHz, $CD_2Cl_2/CD_3OD$ 2:1, -20°C): $\delta_H$ = 0.55 (d, J = 6.5, 3 H, 24-Me); 0.73 (d, J = 7, 3 H, 20-Me); 0.80 (m, 6 H, 18-Me, 8-Ethyl-$CH_3$); 0.98 (d, J = 7, 3 H, 6-Me); 1.02 (d, J = 7, 3 H, 10-Me); 1.06 (m, 2 H, 8-Ethyl-$CH_2$); 1.40-1.49 (m, 3 H, 8-H, 24-H, 22β-H); 1.57 (dd, J = 6.6, 1.5, 3 H, 28-Me); 1.68 (m, 1 H, 20-H); 1.80 (s, 3 H, 12-Me); 1.93 (s, 3 H, 4-Me); 1.96-2.03 (m, 3 H, 18-H, 11-$CH_2$); 2.16 (m, 1 H, 22α-H); 2.40 (m, 1 H, 10-H); 2.66 (m, 1 H, 6-H); 3.12 (dd, J = 10, 0.5, 1 H, 9-H); 3.22 (s, 3 H, 16-OMe); 3.36 (dd, J = 10, 8, 1 H, 25-H); 3.43 (m, 1 H, 21-H); 3.50 (s, 3 H, 2-OMe); 3.55 (m, 1 H, 17-H); 3.68 (dd, J = 10, 2, 1 H, 19-H); 3.85 (dd, J = 9, 1 H, 16-H); 4.42 (m, 1 H, 23-H); 5.17 (verd., 17-H); 5.24 (m, 2H, 26-H, 15-H); 5.55 (dq, J = 15, 6.5, 1 H, 27-H); 5.67 (d, J = 10, 1 H, 5-H); 5.75 (d, J = 11, 1 H, 13-H); 6.41 (s, 1 H, 3-H); 6.62 (dd, J = 15, 11, 1 H, 14-H); 8.18 (d, J

= 8.8, 2 H, 3 -H); 8.46 (d, J = 8.5, 2 H, 2 -H) ppm.

$^{13}C$-NMR (125 MHz, $CD_2Cl_2/CD_3OD$ 2:1, -20°C): $\delta_C$= 8.1 (20-Me); 9.7 (18-Me); 12.0 (8-$CH_2$-$\underline{C}H_3$), 13.6 (24-Me); 14.3 (4-Me); 16.5 (12-Me); 17.1 (6-Me); 17.9 (28-Me); 22.0 (10-Me); 23.1 (8-$\underline{C}H_2$-$CH_3$); 35.4 (C-6); 36.1 (C-10); 37.3 (C-22); 37.8 (C-18); 39.7 (C-20); 41.7 (C-24); 44.3 (C-8); 45.9 (C-11); 56.0 (16-OMe); 59.6 (2-OMe); 69.7 (C-7); 73.9 (C-19); 76.3 (C-21); 76.4 (C-17); 79.9 (C-9); 82.7 (C-16); 82.9 (C-25); 87.6 (C-23); 123.3 (C-13); 125.1 (2 x C-3 ); 127.1 (C-15); 129.8 (2 x C-2 ); 130.1 (C-27); 130.3 (C-26); 131.7 (C-3); 132.0 (C-4); 134.2 (C-14); 141.6 (C-5); 142.2 (C-2); 143.2 (C-12, C-$SO_3$); 151.2 (C-$NO_2$); 166.7 (C-1) ppm.

Beispiel 21: 21,23-Didesoxy-23-epi-azidoconcanolid A

Eine Mischung von 119 mg 23-O-(p-Nitrobenzolsulfonyl)-21-desoxyconcanolid A (0.13 mmol) und 163 mg (2.5 mmol) $NaN_3$ in 16.5 ml DMF werden 45 min bei 40°C gerührt. Nach Zugabe von 15 ml Phosphatpuffer (pH 7, Merck) wird die Lösung mit $CHCl_3$ extrahiert. Nach der üblichen Aufarbeitung wird mehrfach in Toluol aufgenommen, um restliches DMF durch azeotrope Destillation (25 Torr, 50°C) im Rotationsverdampfer zu entfernen. Es folgt eine Säulenchromatographie (EtOAc/Hexan, 1:2), wobei 63 mg (69 %) Produkt und 32 mg Edukt erhalten werden können.

Smp.: 97°C
$C_{39}H_{63}O_8N_3$ (701.90)
$R_f$ = 0.43 (System G)
FAB-MS (positive Ionen): m/e = 723 [$M^+$ + Na]
IR: $\nu$ = 3448, 2966, 2930, 2098, 1695, 1622, 1454, 1379, 1249, 1104, 967$cm^{-1}$
UV (MeOH): $\lambda_{max}$ (e) = 244 (33400); 282 nm (14000)
$[\alpha]^{20}_D$ = - 108.61 (c = 0.62, MeOH)

$^1H$-NMR (500 MHz, $CD_2Cl_2/CD_3OD$ 2:1, -20°C): $\delta_H$ = 0.81 (m, 12 H, 24-Me, 8-Ethyl-$CH_3$, 18-Me, 20-Me); 0.98 (d, J = 7, 3 H, 6-Me); 1.02 (d, J = 6.5, 3 H, 10-Me); 1.10 (m, 2 H, 8-Ethyl-$CH_2$); 1.43 (m, 1 H, 8-H); 1.54 (m, 3 H, 22β-H, 24-H, 20-H); 1.60 (dd, J = 6.5, 1.5, 3 H, 28-Me); 1.88 (s, 3 H, 12-Me); 1.94 (s, 3 H, 4-Me); 1.98 (m, 2 H, 11-$CH_2$); 2.04 (m, 2 H, 22α-H, 18-H); 2.39 (m, 1 H, 10-H); 2.67 (m, 1 H, 6-H); 3.11 (d, J = 10, 5, 1 H, 9-H); 3.22 (s, 3 H, 16-OMe); 3.54 (s, 3 H, 2-OMe); 3.62 (m, 2 H, 19-H, 25-H); 3.68 (m, 2 H, 21-H, 7-H); 3.86 (dd, J = 9, 1 H, 16-H); 3.91 (m, 1 H, 23-H); 5.12 (dd, J = 9, 1, 1 H, 17-H); 5.15 (dd,J = 15,9,1 H, 15-H); 5.23 (ddq,J = 15,8,1, 1H, 26-H); 5.58 (dq, J = 15, 6.5, 1 H, 27-H); 5.68 (d, J = 10, 1 H, 5-H); 5.76 (d, J = 11, 1 H, 13-H); 6.42 (s, 1 H, 3-H); 6.60 (dd, J = 14, 11, 1H, 14-H) ppm.

$^{13}C$-NMR (125 MHz, $CD_2Cl_2/CD_3OD$ 2:1, -20°C): $\delta_C$ = 8.3 (20-Me); 9.9 (18-Me); 12.2 (8-Ethyl-$CH_3$); 14.5 (4-Me); 14.9 (24-Me); 16.6 (12-Me); 17.3 (6-Me); 18.0 (28-Me); 22.0 (10-Me); 23.3 (8-Ethyl-$CH_2$); 34.9 (C-22); 35.7 (C-6); 36.2 (C-10); 38.2 (C-18); 39.8 (C-24, C-20); 44.7 (C-8); 46.2 (C-11); 6.1 (16-OMe); 59.7 (2-OMe); 63.2 (C-23); 70.0 (C-19); 73.6 (C-7); 74.2 (C-21); 76.5 (C-17); 79.9 (C-25); 80.0 (C-9); 83.1 (C-16); 123.6 (C-13); 127.4 (C-15); 129.5 (C-27); 131.2 (C-26); 131.7 (C-3); 132.2 (C-4); 134.3 (C-14); 141.7 (C-5); 142.5 (C-2); 143.4 (C-12); 166.8 (C-1) ppm.

Beispiel 22: 21,23-Didesoxy-23-epi-chlorconcanolid A

Eine Mischung aus 92.7 mg (0.14 mmol) 21-Desoxyconcanolid A und 61.4 mg (0.23 mmol, 1.6 äq.) Triphenylphosphin in 20 ml $CCl_4$ lässt man 24 h bei 100°C rühren. Nach Abkühlen auf Raumtemperatur wird das Lösungsmittel im Rotationsverdampfer entfernt, und der Rückstand durch Säulenchromatographie (Kieselgel, Laufmittel EtOAc/Hexan 1:2) aufgetrennt. Es werden 22.1 mg Chlorid (43 % vom Umsatz) und 43.2 mg Ausgangsprodukt erhalten.

Smp.: 100°C
$C_{39}H_{63}O_8Cl$ (695.37)
$R_f$ = 0.47 (System G)
FAB-MS (positive Ionen): m/e = 694/696 [$M^+$], 716/718 [$M^+$ + Na]
IR: $\nu$ = 3505, 2966, 2875, 1695, 1453, 1358, 1248, 1104, 1005, 967, 762 $cm^{-1}$
UV (MeOH): $\lambda_{max}$ (e) = 244 (35100); 283 nm (15600)
$[a]^{20}_D$ = 18.5 (c = 0.59 MeOH)

$^1H$-NMR (500 MHz, $CD_2Cl_2/CD_3OD$ 2:1, -20°C): $\delta_H$ = 0.82 (m, 12 H, 24-Me, 18-Me, 20-Me, 8-Ethyl-$CH_3$); 0.99 (d, J = 7, 3 H, 6-Me); 1.02 (d, J = 7, 10-Me); 1.11 (m, 2 H, 8-Ethyl-$CH_2$); 1.43 (ddt, J = 11, 8, 3, 1 H, 8-H); 1.60 (dd, J = 6.5, 1.5, 3 H, 28-Me); 1.62 (m, verd., 1 H, 20-H); 1.68 (ddq, J = 10, 6.5, 3, 1 H, 24-H); 1.76 (m, 1 H, 22β-H); 1.88 (s, 3 H, 12-Me); 1.93 (m, verd., 2 H, 11-$CH_2$); 1.94 (s, 3 H, 4-Me); 2.04 (m, 1 H, 18-H); 2.13 (dd, J = 14, 5, 1 H, 22a-H); 2.40 (m, 1 H, 10-H); 2.67 (m, 1 H, 6-H); 3.12 (d, J = 10.5, 1 H, 9-H); 3.22 (s, 3 H, 16-OMe); 3.52 (s, 3 H, 2-OMe); 3.64 (dd, J = 10.5, 1.5, 1 H, 19-H); 3.68 (dd, J = 10, 2.5, 1 H, 7-H); 3.81 (dd, J =

10, 8, 1 H, 25-H); 3.86 (dd, J = 9, 1 H, 16-H); 3.91 (dt, J = 10, 1, 1 H, 21-H); 4.47 (n, 1 H, 23-H); 5.14 (dd, J = 9, 0.5, 1 H, 17-H); 5.15 (dd, J = 15, 9, 1 H, 15-H); 5.26 (ddq, J = 15, 8, 1, 1 H, 26-H); 5.60 (dq, J = 15, 6, 1 H, 27-H); 5.68 (d, J = 10, 1 H, 5-H); 5.80 (d, J = 11, 1 H, 13-H); 6.42 (s, 1 H, 3-H); 6.62 (dd, J = 15, 11, 1 H, 14-H) ppm.

$^{13}$C-NMR (125 MHz, $CD_2Cl_2/CD_3OD$ 2:1, -20°C): $\delta_C$ = 8.3 (20-Me); 9.8 (18-Me); 12.0 (8-$CH_2$-$\underline{C}H_3$); 14.3 (4-Me); 15.7 (24-Me); 16.5 (12-Me); 17.1 (6-Me); 18.0 (28-Me); 21.8 (10-Me); 23.1 (8-$\underline{C}H_2$-$CH_3$); 35.4 (C-6); 36.3 (C-10); 38.0 (C-18); 38.9 (C-22); 39.4 (C-20); 40.5 (C-24); 44.4 (C-8); 45.8 (C-11); 56.0 (16-OMe); 59.7 (2-OMe); 65.0 (C-23); 69.9 (C-19); 73.0 (C-21); 74.2 (C-7); 76.4 (C-17); 78.8 (C-25); 79.8 (C-9); 82.9 (C-16); 123.4 (C-13); 127.2 (C-15); 129.5 (C-27); 130.8 (C-26); 131.5 (C-3); 132.1 (C-4); 134.0 (C-14); 141.3 (C-5); 142.3 (C-2); 143.0 (C-12); 166.6 (C-1) ppm.

Beispiel 23: Oxidation von 21-Desoxyconcanolid A zu 21-Desoxyconcanolid A-23-keton

138 mg 21-Desoxyconcanolid A (0.20 mmol) werden in 20 ml trockenem $CH_2Cl_2$ gelöst und mit 87.5 mg (0.65 mmol, 3.25 äq.) 4-Methylmorpholin-4-oxid Monohydrat und 6.2 mg (0.02 mmol, 0.1 äq.) Tetrapropylammonium-perruthenat versetzt und bei Raumtemperatur 2 h gerührt. Die Reaktionsmischung wird ohne weitere Aufarbeitung durch Chromatographie an Kieselgel (Laufmittel EtOAc/Hexan, 1:1) getrennt. Man erhält 45.2 mg 21-Desoxyconcanolid A-9/23-diketon (39 % vom Umsatz), 20.9 mg 21-Desoxyconcanolid A-23-keton (17 % vom Umsatz) und 16 mg Ausgangsprodukt. Das Produktspektrum lässt sich durch Variation der Reaktionstemperatur steuern. Bei 0°C wird nur 21-Desoxyconcanolid A-23-keton in geringer Ausbeute erhalten (< 20 %), bei Raumtemperatur tritt zusätzlich das 21-Desoxyconcanolid A-9, 23-diketon auf, ohne dass sich die Ausbeute an 21-Desoxyconcanolid A-23-keton erhöht.

21-Desoxyconcanolid A-23-keton:
Smp.: 98°C
$C_{39}H_{62}O_9$ (674.91)
$R_f$ = 0.57 (System F)
FAB-MS (positive Ionen): m/e = 696 [$M^+$ + Na]
IR: $\nu$ = 3451, 2969, 2931, 1709, 1621, 1455, 1356, 1248, 1103, 967cm$^{-1}$
UV (MeOH): $\lambda_{max}$ (e) = 245 (26200); 282 nm (11500)
$[\alpha]^{20}_D$ = + 3.7 (c = 0.27 MeOH)

$^1$H-NMR (500 MHz, $CD_2Cl_2/CD_3OD$ 2:1, -20°C): $\delta_H$ = 0.83 (m, 12 H, 20-Me, 18-Me, 20-Me, 8-Ethyl-$CH_3$); 0.99 (d, J = 7, 3 H, 6-Me); 1.02 (d, J = 7, 3 H, 10-Me); 1.10 (m, 2 H, 8-Ethyl-$CH_2$); 1.42 (m, 1 H, 8-H); 1.63 (dd, J = 6.5, 1.5, 3 H, 28-Me); 1.80 (m, 1 H, 20-H); 1.89 (s, 3 H, 12-Me); 1.94 (s, 3 H, 4-Me); 2.00-2.09 (m, 3 H, 18-H, 11-$CH_2$); 2.31 (m, 2 H, 24-H, 22$\beta$-H); 2.40 (m, 1 H, 10-H); 2.52 (dd, J = 9, 2.5, 1 H, 22$\alpha$-H); 2.68 (m, 1 H, 6-H); 3.12 (d, J = 10, 1 H, 9-H); 3.22 (s, 3 H, 16-OMe); 3.53 (s, 3 H, 2-OMe); 3.59 (dd, J = 11, 8, 1 H, 25-H); 3.67 (m, 3 H, 7-H, 19-H, 21-H); 3.87 (dd, J = 9, 1 H, 16-H); 5.12 (dd, J = 9, 1, 1 H, 17-H); 5.15 (dd, 15, 9, 1 H, 15-H); 5.40 (ddq, J = 10, 6.5, 2, 1 H, 27-H); 5.63 (dq, J = 6.5, 1 H, 26-H); 5.69 (d, J = 10, 1 H, 13-H); 5.76 (d, J = 11, 1 H, 5-H); 6.42 (s, 1 H, 3-H); 6.63 (d, J = 15, 11, 1 H, 14-H) ppm.

$^{13}$C-NMR (125 MHz, $CD_2Cl_2/CD_3OD$ 2:1, -20°C): $\delta_C$ = 7.8 (20-Me); 9.7 (24-Me, 18-Me); 12.0 (8-Ethyl-$CH_3$); 14.4 (4-Me); 16.4 (12-Me); 17.2 (6-Me); 18.0 (28-Me); 22.0 (10-Me); 23.0 (8-Ethyl-$CH_2$); 35.4 (C-6); 36.0 (C-10); 37.7 (C-18): 40.6 (C-20); 44.3 (C-8); 46.0 (C-11); 46.7 (C-22); 50.4 (C-24); 56.0 (16-OMe); 59.6 (2-OMe); 69.7 (C-19); 73.8 (C-21); 76.3 (C-17); 78.7 (C-7); 79.8 (C-9); 82.6 (C-16); 84.6 (C-25); 123.3 (C-13); 127.0 (C-15); 130.1 (C-26); 130.6 (C-27); 131.7 (C-3); 132.0 (C-4); 134.6 (C-14); 141.6 (C-5); 142.1 (C-12); 143.3 (C-2); 166.8 (C-1); 210.7 (C-23) ppm.

21-Desoxyconcanolid A-9, 23-diketon:
Smp.: 100°C
$C_{39}H_{60}O_9$ (672.90)
$R_f$ = 0.71 (System F)
FAB-MS (positive Ionen): m/e = 694 [$M^+$ + Na]
IR: $\nu$ = 3454, 2970, 2932, 1701, 1623, 1454, 1357, 1248, 1104, 968 cm$^{-1}$
UV (MeOH): $\lambda_{max}$ (e) = 244 (34400); 278 nm (15100)
$[\alpha]^{20}_D$ = -53.75 (c = 0.4 MeOH)

$^1$H-NMR (500 MHz, $CD_2Cl_2/CD_3OD$ 2: 1, -20°C): $\delta_H$ = 0.71 (t, J = 7, 3 H, 8-Ethyl-$CH_3$); 0.81 (d, J = 7, 3 H, 18-Me); 0.84 (d, J = 7, 3 H, 24-Me); 0.85 (d, J = 7, 3 H, 20-Me); 0.97 (d, J = 7, 3 H, 6-Me); 1.09 (d, J = 7, 3 H, 10-Me); 1.11 (m, verd., 1 H, 8-Ethyl-$CH_2$, $\beta$-H); 1.44 (ddt, J = 13.5, 11, 7, 1 H, 8-Ethyl-$CH_2$, $\alpha$-H); 1.64 (dd, J = 6.5, 1.5, 3 H, 28-Me); 1.80 (m, 1 H, 20-H); 1.94 (s, 3 H, 12-Me); 1.97 (s, 3 H, 4-Me); 2.05 (m, 1 H, 18-H); 2.31 (m, 3 H, 24-H, 22$\beta$-H, 11$\beta$-H); 2.42 (dd, J = 16, 8, 1 H, 11$\alpha$-H); 2.52 (dd, J = 14, 2.5, 1 H, 22$\alpha$-H); 2.61 (ddd, J = 10, 7, 2.5, 1 H, 6-H); 2.78 (dd, J = 10, 2.5, 1 H, 8-H); 2.93 (m, 1 H, 10-H); 3.24 (s, 3 H, 16-OMe); 3.45 (dd, J =

10, 2.5, 1 H, 19-H); 3.55 (s, 3 H, 2-OMe); 3.60 (m, 2 H, 21-H, 25-H); 3.67 (m, 1 H, 7-H); 3.89 (dd, J = 9, 1 H, 16-H); 5.19 (dd, J = 9, 1, 1 H, 17-H); 5.24 (dd, J = 14.5, 9, 1 H, 25-H); 5.41 (ddq, J = 15, 8, 1.5, 1 H, 26-H); 5.63 (dq, J = 15, 6.5, 1 H, 27-H); 5.73 (d, J = 9.5, 1 H, 5-H); 5.93 (d, J = 10.5, 1 H, 13 H); 6.50 (s, 1 H, 3-H); 6.67 (dd, J = 15, 11, 1 H, 14-H) ppm.

$^{13}C$-NMR (125 MHz, $CD_2Cl_2/CD_3OD$ 2:1, -20°C): $\delta_C$ = 7.8 (20-Me); 9.7 (24-Me); 9.8 (18-Me); 12.2 (8-Ethyl-$CH_3$); 14.4 (4-Me); 15.9 (12-Me); 16.5 (6-Me); 18.0 (10-Me, 28-Me); 20.8 (8-Ethyl-$CH_2$); 35.4 (C-6); 37.8 (C-18); 40.5 (C-20); 42.0 (C-11); 46.0 (C-10); 46.7 (C-22); 50.4 (C-24); 54.2 (C-8); 56.0 (16-OMe); 59.7 (2-OMe); 69.6 (C-7); 75.9 (C-19); 76.2 (C-17); 78.7 (C-21); 82.7 (C-16); 84.5 (C-25); 124.7 (C-13); 127.8 (C-15); 130.0 (C-27); 130.5 (C-26); 131.6 (C-3); 133.0 (C-4); 133.9 (C-14); 140.0 (C-5); 141.5 (C-2); 142.3 (C-12); 166.5 (C-1); 210.6 (C-23); 218.1 (C-9) ppm.

**Patentansprüche**

1. Verwendung einer Verbindung der Formel

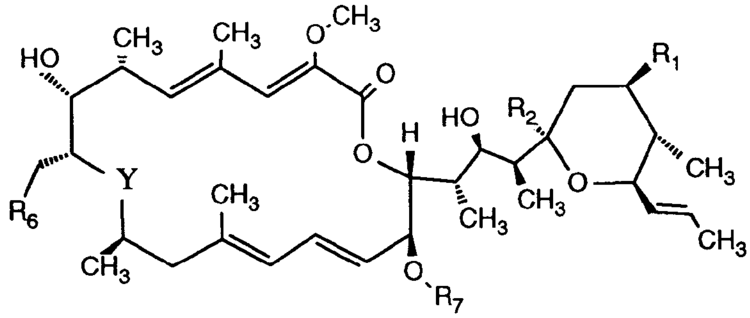

(I),

worin $R_1$ Hydroxy, 2-Hydroxyethoxy, $C_1$-$C_4$-Alkoxy, $C_1$-$C_4$-Alkanoyloxy, Halogen, Amin, Azid, eine Keto, p-Nitrobenzolsulfonyl oder eine Pyranyloxy-Gruppe der Formel

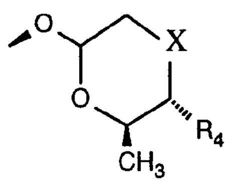

bedeutet, worin X eine Gruppe der Formel

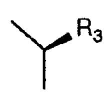

oder

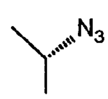

ist, worin $R_3$ für Hydroxy, $C_1$-$C_4$-Alkanoyloxy oder organisches Sulfonyloxy steht, und
$R_4$ Hydroxy, $C_1$-$C_4$-Alkanoyloxy oder Carbamoyloxy ist,
$R_2$ Wasserstoff, Hydroperoxy, Hydroxy oder $C_1$-$C_4$-Alkoxy darstellt, Y eine Gruppe der Formel

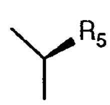

oder

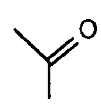

ist, worin $R_5$ Hydroxy oder $C_1$-$C_4$-Alkanoyloxy bedeutet, und
$R_6$ und $R_7$ unabhängig voneinander Wasserstoff oder Methyl darstellen, zur Herstellung eines Arzneimittels, welches für die Prophylaxe und Behandlung von Krankheiten, die auf die Inhibition der Osteoklasten-Protonenpumpe ansprechen, geeignet ist.

2. Verwendung einer Verbindung der Formel (I) gemäss Anspruch 1, worin $R_1$ Hydroxy, Methoxy, $NH_3$, Chlor, Brom, eine Keto, p-Nitrobenzolsulfonyl oder eine Pyranyloxy-Gruppe der Formel

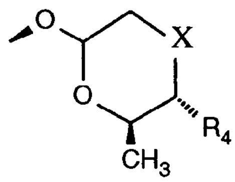

bedeutet, worin $R_4$ Hydroxy, Acetoxy oder Carbamoyloxy ist und X eine Gruppe der Formel

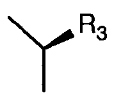

oder

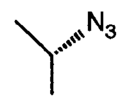

ist, worin $R_3$ für Hydroxy, Acetoxy oder unsubstituiertes oder durch $C_1$-$C_4$-Alkyl, Halogen oder Nitro einfach substituiertes Benzolsulfonyloxy steht,
$R_2$ Wasserstoff oder Hydroxy darstellt, Y eine Gruppe der Formel

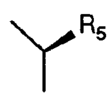

oder

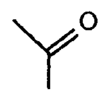

ist, worin $R_5$ Hydroxy oder Acetoxy bedeutet, und $R_5$ Methyl darstellt.

3. Verwendung einer Verbindung der Formel (I) gemäss Anspruch 1, worin $R_1$ Hydroxy, Methoxy, $NH_3$, Chlor, eine Keto oder eine Pyranyloxy-Gruppe der Formel

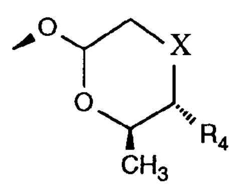

bedeutet, worin $R_4$ Hydroxy oder Carbamoyloxy ist und X eine Gruppe der Formel

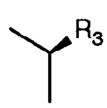

ist, worin $R_3$ Hydroxy oder Acetoxy bedeutet,
$R_2$ Wasserstoff oder Hydroxy darstellt, und Y eine Gruppe der Formel

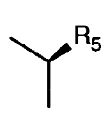

darstellt und $R_5$ Hydroxy oder Acetoxy bedeutet.

4. Verwendung von Concanamycin A gemäss Anspruch 1.

5. Verwendung von Concanolid A gemäss Anspruch 1.

6. Verwendung von Concanamycin B gemäss Anspruch 1.

7. Verwendung von Concanolid B gemäss Anspruch 1.

8. Verwendung von Concanamycin C gemäss Anspruch 1.

9. Verwendung von 21-Desoxy-concanolid A gemäss Anspruch 1.

10. Verwendung von 21-Desoxy-concanolid B gemäss Anspruch 1.

11. Verwendung von 23-O-Methyl-concanolid A gemäss Anspruch 1.

12. Verwendung von 3',9-Di-O-acetyl-concanamycin A gemäss Anspruch 1.

13. Verwendung von 16-Desmethyl-21-desoxyconcanolid A, 21,23-Di-desoxy-23-epi-azidoconcanolid A, 23-O-(p-Nitrobenzolsulfonyl)-21-desoxyconcanolid A, 21-Desoxyconcanolid A-23-keton, 21-Desoxyconcanolid A-9, 23-diketon und/oder 21,23-Didesoxy-23-epi-chlorconcanolid A gemäss Anspruch 1.

14. Verbindung der Formel

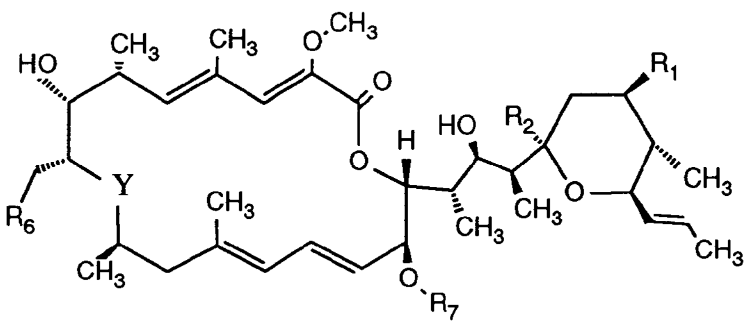

(I),

worin $R_6$ und $R_7$ unabhängig voneinander jeweils Wasserstoff oder Methyl ist und

- worin $R_1$ Hydroxy, Halogen, Amin, Azid, p-Nitrobenzolsulfonyl oder Keto ist, $R_2$ Wasserstoff bedeutet und Y eine Gruppe der Formel

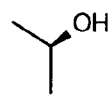

darstellt, oder

- worin $R_1$ Halogen, Amin, Azid, Keto, p-Nitrobenzolsulfonyl oder Pyranyloxy-Gruppe der Formel

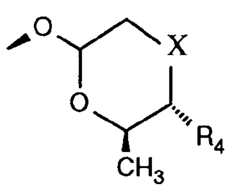

bedeutet, worin $R_4$ Carbamoyloxy ist und X eine Gruppe der Formel

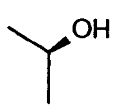

ist, $R_2$ für Hydroxy steht und Y eine Gruppe der Formel

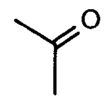

darstellt, oder

- $R_1$ Halogen, Amin, Azid, 2-Hydroxyethoxy, eine Keto, p-Nitrobenzolsulfonyl oder eine Pyranyloxy-Gruppe der Formel

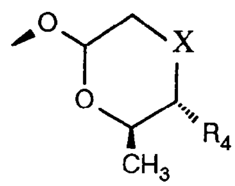

bedeutet, worin $R_4$ Hydroxy oder Carbamoyloxy ist und X eine Gruppe der Formel

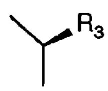

oder

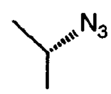

ist, worin $R_3$ für unsubstituiertes oder durch $C_1$-$C_4$-Alkyl, Halogen oder Nitro einfach substituiertes Benzolsulfonyloxy steht,
$R_2$ Wasserstoff, Hydroperoxy oder Hydroxy darstellt und Y eine Gruppe der Formel

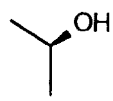

ist, oder

- $R_1$ Halogen, Amin, Azid, eine Ketogruppe, p-Nitrobenzolsulfonyl, $C_1$-$C_4$-Alkoxy oder $C_1$-$C_4$-Alkanoyloxy ist, $R_2$ Hydroxy oder $C_1$-$C_4$-Alkoxy darstellt und Y eine Gruppe der Formel

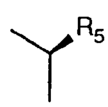

darstellt und $R_5$ $C_1$-$C_4$-Alkanoyloxy bedeutet.

**15.** Verbindung der Formel (I) gemäss Anspruch 14, worin

- $R_1$ Hydroxy, Azid oder Chlor ist, $R_2$ Wasserstoff bedeutet und Y eine Gruppe der Formel

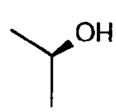

darstellt, oder

- $R_1$ eine Pyranyloxy-Gruppe der Formel

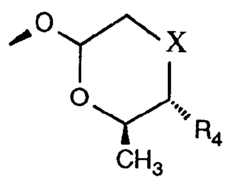

bedeutet, worin $R_4$ Carbamoyloxy ist und X eine Gruppe der Formel

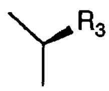 oder 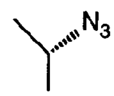

ist, worin $R_3$ durch Nitro einfach substituiertes Benzolsulfonyloxy ist, $R_2$ Hydroxy darstellt und Y eine Gruppe der Formel

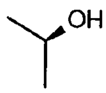

ist, oder

- $R_1$ Acetoxy ist, $R_2$ Hydroxy darstellt und Y eine Gruppe der Formel

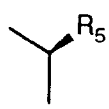

darstellt und $R_5$ Acetoxy bedeutet.

**16.** 21-Desoxy-concanolid A gemäss Anspruch 14.

**17.** 16-Desmethyl-21-desoxyconcanolid A gemäss Anspruch 14.

**18.** 21,23-Didesoxy-23-epi-azidoconcanolid A gemäss Anspruch 14.

**19.** 21,23-Didesoxy-23-epi-chlorconcanolid A gemäss Anspruch 14.

**20.** 23-O-(p-Nitrobenzolsulfonyl)-21-desoxyconcanolid A gemäss Anspruch 14.

**21.** 21-Desoxyconcanolid A-23-keton gemäss Anspruch 14.

**22.** 21-Desoxyconcanolid A-9, 23-diketon gemäss Anspruch 14.

**23.** Pharmazeutische Zusammensetzung enthaltend eine Verbindung der Formel (I) gemäss Anspruch 14.

**24.** Eine Verbindung der Formel (I) gemäss Anspruch 14 zur Anwendung in einem Verfahren zur prophylaktischen oder therapeutischen Behandlung des menschlichen oder tierischen Körpers.

Europäisches Patentamt

# EUROPÄISCHER RECHERCHENBERICHT

Nummer der Anmeldung

EP 94810460.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 6) |
|---|---|---|---|
| A | WO - A - 91/06 296 (AKTIEBOLAGET ASTRA) * Ansprüche 1,2,7-11,16-30 * | 1-24 | C 12 P 17/08<br>C 07 D 313/00<br>C 07 D 407/06<br>A 61 K 31/335<br>A 61 K 31/365 |
| D,A | BIOCHEMISTRY, Band 32, 1993 (New York) S. DRÖSE et al. "Inhibitory Effect of Modified Bafilomycins and Concanamycins on P- and V-Type Adenosintriphosphatases" Seiten 3902-3906 * Gesamt * | 1-24 | |
| A | PATENT ABSTRACTS OF JAPAN, unexamined applications, C Field, Nr. 373, 14. Juli 1993 THE PATENT OFFICE JAPANESE GOVERNMENT Seite 19 C 1083; & JP-A-05 59 046 (AJINOMOTO) | 1-24 | RECHERCHIERTE SACHGEBIETE (Int Cl6) |
| D,A | THE JOURNAL OF ANTIBIOTICS, Band 37, Nr. 11, November 1984 (Tokyo) H. KINASHI et al. "Isolation and Characterization of Concanamycins A,B and C" Seiten 1333-1335 * Seite 1333 * | 14-21 | C 12 P<br>C 07 D<br>A 61 K |

Der vorliegende Recherchenbericht wurde für alle Patentansprüche erstellt.

| Recherchenort | Abschlußdatum der Recherche | Prüfer |
|---|---|---|
| WIEN | 15-11-1994 | WOLF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82